# EUROPEAN PATENT APPLICATION

(11) **EP 4 631 570 A2**
(43) Date of publication of application: **15.10.2025**
(21) Application number: 25194793.3
(22) Date of filing: 09.07.2021
(51) Int. Cl.: A61P 9/00

(54) **MICRORNA-TARGETED THERAPY FOR CARDIAC REPAIR**

(30) Priority: 10.07.2020 EP 20185192
(62) Divisional of application: 21742376.3
(71) Applicant: Charité - Universitätsmedizin Berlin, 10117 Berlin (DE)
(72) Inventor: Jakob, Philipp, 7000 Chur (CH); Landmesser, Ulf, 14195 Berlin (DE); Renikunta, Harsha Vardhan, 55128 Mainz (DE)
(74) Representative: Gulde & Partner

(57) **Abstract**

The invention is directed to a miRNA with a RNA sequence comprising or consisting of hsa-miR-4784 with SEQ ID NO: 6, or a member of the miR-4784 family, or a primary transcript thereof, a precursor thereof, a mimic thereof or a combination for use as a medicament, preferably for use in treatment of a cardiac disease associated with loss of cardiac myocytes.

## Description

In adults, cardiac injury results in loss of cardiomyocytes (CM) and subsequent scar formation with heart failure symptoms. Despite progress made over the last decades, heart failure remains the most significant health issue and the leading cause of death worldwide. In 2015, 110 million cases are estimated to suffer from ischemic heart disease (IHD), with approximately 9 million deaths due to IHD. In addition, about 38 million patients suffer from heart failure that is associated with a high mortality.

As regenerative mechanisms of the cardiac muscle are limited or mostly absent, human hearts are prone to hypertrophic remodeling and fibrotic scarring.

Current therapeutic approaches focus primarily on catheter-based or surgical re-perfusion strategies to minimize loss of contractile tissue and medical therapies to prevent adverse cardiac remodeling. However, the damaged myocardium cannot be cured and a therapy that stimulates cardiomyocyte proliferation to re-muscularize the heart does not exist.

Despite advances in technical and drug-based technologies, the previously observed decrease in cardiovascular mortality has reached a plateau, even in high-income regions.

In order to provide improved approaches, microRNAs (miRNAs) were tested for induction of cardiomyocyte proliferation. MicroRNAs are small noncoding RNAs that regulate gene expression at the post-transcriptional level. Repression and/or silencing of gene expression by microRNAs takes place by base-pairing to at least partially complementary sequences present mostly in 3' UTRs of target messenger RNAs (mRNAs). This results in translational repression, mRNA degradation, or both. The microRNA seed sequence is generally situated at positions 2-8 from the microRNA 5'-end. The seed sequence is the primary specificity determinant for target selection. The small size of the seed sequence means that a single microRNA may regulate many, even hundreds, different genes.

MicroRNAs are genome-encoded sequences. They are generally transcribed by the RNA polymerase II into so called primary microRNAs (pri-microRNAs). Two endonucleases of the RNAse III family then process the pri-microRNAs sequentially in the nucleus. Drosha processes the pri-microRNA into a precursor microRNA (pre-microRNA) of approximately 60-80 nucleotides, after which the pre-microRNA is further processed, in the cytoplasm, by Dicer to form a duplex containing two strands, of about 19-23 nucleotides. The microRNA duplex is then unwound, and the mature microRNA is incorporated into the RNA-induced silencing complex (RISC), containing, among others, Argonaute and GW182 proteins essential for the silencing by microRNAs. The reference repository of published microRNA sequences is publicly accessible (miRBase; www.mirbase.org).

Only a few microRNAs have been so far clearly implicated in cardiomyocyte proliferation, including among others miR-1, miR-133 and, more recently, members of the miR-15 family. Overexpression of miR-1 in the embryonic heart is known to inhibit cardiomyocyte proliferation, which was linked to the repression of Hand2, a transcription factor required for cardiac growth during embryogenesis. MiR-133 inhibits proliferation of cardiomyocytes through the repression of SRF and cyclin D2, two essential regulators of muscle cell differentiation. Finally, it is known that the miR-15 family may regulate the post-natal mitotic arrest of mouse cardiomyocytes, by means of a downregulation of the expression of Chek1.

EP 2842577 A1 is a patent application describing miR-148a, miR-148b, miR-152 & miR-373 as well as variants, analogs and precursors thereof in cardiomyocytes. WO 2018183997 describes miR-302 in cardiomyocytes. EP 3143123 B1 describes the micro-mRNA of the miR-302-367-cluster in the context of the developing heart. US 10,337,002 B2 describes hsa-miR-590-3p and hsa-miR-199a-3p and a primary transcript precursor.

WO 2017/210735 A1 and WO 2013/134416 A1 disclose micro-mRNA which are not related to cardiovascular diseases. Pathophysiological and biological processes modulated by miRNAs differ significantly in different cells and different organs. Indeed, the prior art documents are silent on miRNAs which can be used for treatment in cardiovascular diseases.

All in all, there is a need to provide additional treatments and regenerative strategies to increase cell-cycling of human cardiomyocytes with subsequent regeneration of the heart muscle.

### DESCRPTION OF THE INVENTION:

According to an aspect of the invention, a miRNA with a RNA sequence is described which comprises or consists of a sequence having at least 80%, more preferably at least 90%, even more preferably at least 95%, and most preferably being identical to one of the following sequences:
hsa-miR-515-3p with SEQ ID NO: 1, or
hsa-miR-519e-3p with SEQ ID NO:2, or
a member of the miR-517 family, specifically hsa-miR-517c-3p with SEQ ID NO: 3 or hsa-miR-517a-3p with SEQ ID NO: 4,
or a primary transcript thereof, a precursor thereof, a mimic thereof or a combination thereof for use as a medicament.

According to a preferred embodiment, the miRNA is for use in treatment of a cardiac disease associated with loss of (functional) cardiac myocytes. According to a more preferred embodiment, the cardiac disease is selected from myocardial infarction, ischemic and non-ischemic cardiomyopathy, congestive heart failure, hypertrophic cardiomyopathy, dilated cardiomyopathy, myocarditis and chronic heart failure.

The term "miRNA" according to the invention refers to a RNA sequence comprising or consisting of hsa-miR-515-3p with SEQ ID NO: 1, hsa-miR-519e-3p with SEQ ID NO:2, a member of the miR-517 family, specifically hsa-miR-517c-3p with SEQ ID NO: 3 or hsa-miR-517a-3p with SEQ ID NO: 4, or a primary transcript thereof, a precursor thereof, a mimic thereof or a combination thereof.

According to a preferred embodiment of the invention, the miRNA according to the invention is described for use as a medicament for cardiac repair in patients with myocardial injury caused due to the loss of cardiomyocytes. In particular, the miRNA according to the invention is described for use as a medicament for patients with heart failure and heart disease caused or accompanied by loss of cardiomyocytes.

In the context of the present invention, the term "patient" comprises humans and animals, and preferably refers to humans. Likewise, the term "treatment" encompasses treatment of humans and animals, preferably humans.

The microRNAs according to the invention are capable of inducing proliferation in cardiomyocytes in vitro, in particular are capable of inducing proliferation of cardiomyocytes of an animal subject, more in particular in different animal subjects, even more in particular in humans.

The present invention also comprises primary transcripts, precursors and mimics of the microRNAs herein disclosed. The concepts of miRNA primary transcript, precursor and mimic are well-known in the art.

Modifications of microRNA backbone or synthetic nucleic acids anyhow mimicking natural microRNA function are also provided in the present invention. These modifications can be made according to techniques, which are well known to the skilled person, on condition that said modifications do not alter the function of the microRNAs of the present invention. Such modifications, include, but are not restricted to, substitution of non-bonding oxygen atoms in the phosphate group, introduction of an alkyl group in the sugar molecule of nucleotides, inclusion of extra bonds connecting carbon or oxygen atoms in the sugars of nucleotides (for example, the LNA technology), and the like. As stated above, these modifications are well-known to the skilled person and do not require specific further disclosure.

The microRNAs of the present invention are able to increase cardiac myocytes mitosis, cell division (cytokinesis) and cell number in vitro, as demonstrated in the Example section.

According to a preferred embodiment, the miRNA according to the invention is described for use as a medicament for cardiac repair in patients with heart failure due or accompanied by loss of cardiomyocytes, in particular one or more selected from the list of acute coronary syndrome, myocardial infarction and cardiomyopathies.

According to a preferred embodiment, the miRNA according to the invention is described for use as a medicament for patients who survived a heart attack.

According to a preferred embodiment, the miRNA according to the invention is described for use as a medicament in a patients with heart failure, in particular ischemic cardiomyopathy due to myocardial infarction or cardiac dysfunction.

In the embodiments of the present invention relating the microRNAs as medicaments, in particular for the treatment of heart diseases associated with a loss of cardiomyocytes (consequences of myocardial infarction, cardiomyopathy of ischemic and non-ischemic origin, myocarditis and heart failure), they can be administered to a subject suffering from said disease by conventional methods with the specific objective of inducing cardiac regeneration by stimulating proliferation of cardiomyocytes and other cells contributing to cardiac repair, such as endothelial and immune cells.

Conveniently, said medicament is in the form of a preparation for parenteral, intracoronary, intravenous or intracardiac administration, but other forms are equally suitable for carrying out the present invention. The person skilled in the art will decide the effective time of administration, depending on the patient's conditions, degree of severity of the disease, response of the patient and any other clinical parameter within the general knowledge of this matter.

According to a preferred embodiment, the miRNA is for administration via gene therapy, liposomal nanoparticle and/or a cardiotropic virus.

According to another aspect, a vector is described for use as a medicament, preferably for use in treatment of a cardiac disease associated with loss of cardiac myocytes, wherein said vector comprises at least a miRNA according to the invention, or a DNA segment coding for at least one of said miRNA according to the invention.

According to a preferred embodiment, the vector is an adeno-associated vector (AAV) of any capsid serotype, either natural, such as, but not restricted to, AAV1, AAV2, AAV6, AAV8, AAV9, AAV10, or a retrovirus, a lentivirus or a polymeric vector or a dendrimer-based vector or a inorganic or lipid nanoparticle or cell-derived membrane vesicles or scaffold-based delivery systems (hydrogels, electrospun fibers).

According to an embodiment of the invention, the miRNA according to the invention is for use as a medicament for intramyocardial injection into the peri-infarct region, in particular after coronary intervention.

According to a preferred embodiment of the invention, the miRNA according to the invention is for use as a medicament for reducing scar formation and preserve or improve contractility of the myocardium.

According to a preferred embodiment of the invention, the miRNA according to the invention is for use as a medicament in patients with a cardiac dysfunction or reduced LV-function due to heart failure, in particular ischemic cardiomyopathy. Specifically, as the miRNAs of the invention increase proliferation of cardiomyocytes under transient hypoxic conditions, heart failure associated with ischemia (such as ischemic cardiomyopathy, myocardial infarction) is a preferred treatment.

According to an aspect, the miRNA according to the invention is for use in vivo, ex vivo or in vitro for generation and proliferation of cardiomyocytes, myocardial patches and tissue replacement therapy.

According to a preferred embodiment of the invention, the miRNA according to the invention is for use as a medicament for administration which takes place orally, parenterally, perlingually, intraarterial, intracoronary or intravenously or via a stent that is used for diseases related to atherosclerosis.

According to a more preferred embodiment of the invention, the miRNA according to the invention is for use as a medicament for administration which takes place via a stent, in particular a medicament that is released into the post-stenotic or post-occluded ischemic myocardium in patients with cardiovascular disease and myocardial infarction.

Furthermore, a dedicated catheter systems for transendocardial injections can be used. For instance, a catheter-based myocardial injection system may be used for delivery of miRNA wherein the carrier for the miRNA may be an adeno-associated vector (AAV) of any capsid serotype, either natural (such as, but not restricted to, AAV1, AAV2, AAV8, AAV9) or a retrovirus, a lentivirus or a polymeric vector or a dendrimer-based vector or a inorganic or lipid nanoparticle or cell-derived membrane vesicles or scaffold-based delivery systems (for instance hydrogels, electrospun fibers).

According to a more preferred embodiment of the invention, the miRNA according to the invention is for use as a medicament for administration which takes place intravenously, in particular via a miRNAs that are chemically modified and delivered through lipoplexes, lipid nanoparticle, polymers or extracellular vesicles which comprises the miRNA according to the invention.

According to a preferred embodiment of the invention, the miRNA according to the invention is for use as a medicament which takes place intravenously, in particular via a cardiotropic virus, in particular one that do not integrate into the cell genome, for instance AAV6.

According to a preferred embodiment of the invention, the miRNA according to the invention is for use as a medicament for administration which takes place via intramyocardial injection.

According to a further aspect, a pharmaceutical composition comprising at least a miRNA, as descried above, or a vector, as described above, and at least one pharmaceutically acceptable excipient.

According to a preferred embodiment, the pharmaceutical composition is for use in treatment of a cardiac disease associated with loss of cardiac myocytes. In particular, the cardiac disease is selected from myocardial infarction, ischemic and non-ischemic cardiomyopathy, congestive heart failure, hypertrophic cardiomyopathy, dilated cardiomyopathy, myocarditis and chronic heart failure.

The pharmaceutical compositions will contain at least one of the following: synthetic RNA corresponding to the microRNA of the present invention or its primary transcript or precursor, DNA coding for said microRNA, DNA coding for a primary transcript or precursor for said RNA such as the microRNA is produced inside the cells containing this DNA. The microRNAs (or primary transcript or precursor) of the present invention or the corresponding coding DNAs can be administered together with lipidic molecules such as cationic lipids, or peptides, or in the context of polymeric scaffolds, which can facilitate their delivery, according to the art. Another method to administer such microRNAs or their corresponding DNAs is by means of a suitable vector known for the administration of RNA or DNA. A preferred vector is the adeno-associated vector (AAV) of any capsid serotype, either natural (such as, but not restricted to, AAV1, AAV2, AAV8, AAV9) or artificial, a well-known viral vector for administration of DNA in vivo. All these methods and formulation to administer the above synthetic RNA corresponding to the microRNA of the present invention, DNA coding for said microRNA, DNA coding for a primary transcript or precursor for said RNA such as the microRNA is produced inside the cells containing this DNA are conventional and well known in the art and do not need further explanation.

Injection is a further preferred administration route. The person skilled in the art can decide to administer microRNAs by means of any conventional pharmaceutical composition.

The administration regime, dosage and posology may be determined by the physician according to his experience, the disease to be treated and the patient's conditions.

Depending on the administration route chosen, the compositions will be in solid or liquid form, suitable for oral, parenteral, intravenous or intra-arterial administration. Gene therapy is a further embodiment. The compositions according to the present invention contain, along with the active ingredient, at least one pharmaceutically acceptable vehicle or excipient. These may be particularly useful formulation coadjuvants, e.g. solubilizing agents, dispersing agents, suspension agents, and emulsifying agents.

The active agents for use in the present invention can be administered as a medicament, in particular as a pharmaceutical composition. The pharmaceutical composition comprises at least one active agent of the present invention with a suitable carrier. Different routes and techniques for administration can be applied, for instance parenteral techniques such as intravenous, intracardiac, and intra-arterial injections, catheterizations and the like. Average quantities of the active agent may vary and may be adapted suitably.

According to a further aspect, a method for the in vitro expansion of cardiomyocytes derived from stem cells is described, the method comprising the step of endogenous overexpression with at least one miRNA in said cells, as described above.

According to a further aspect, a method for stimulating proliferation of cardiomyocytes in vitro is described, the method comprising the step of endogenous overexpression with at least one miRNA in said cells as described above.

According to an additional aspect of the invention, the use of an miRNA according to the invention is described in vitro or ex vivo in the promotion of cardiomyocyte proliferation and in the expansion of cardiomyocytes derived from embryonic stem (ES) cells, induced pluripotent (iPS) cells or stem cells obtained by other procedures.

Under a standard nomenclature system for miRNAs, names are assigned to experimentally confirmed miRNAs wherein the prefix "miR" is followed by a dash and a number. A capitalized "miR-" refers to the mature form of the miRNA. Further, the term 'hsa-' may be of interest: The first three letters signify the organism. For humans for instance, those letters are 'hsa'.

According to the invention, the miRNA of the invention as referred to above is intended also for use as a medicament. In this context, the miRNA according to the invention is described for use in a medical treatment in general.

In the following, the invention is further described by way of examples.

### EXAMPLES:

**Table 1: miRNA sequences:**

| **Name** | **Sequence** | **SEQ ID NO** |
|---|---|---|
| hsa-miR-515-3p | GAGUGCCUUCUUUUGGAGCGUU | SEQ ID NO: 1 |
| hsa-miR-519e-3p | AAGUGCCUCCUUUUAGAGUGUU | SEQ ID NO: 2 |
| hsa-miR-517c-3p | AUCGUGCAUCCUUUUAGAGUGU | SEQ ID NO: 3 |
| hsa-miR-517a-3p | AUCGUGCAUCCCUUUAGAGUGU | SEQ ID NO: 4 |
| hsa-miR-371a-3p | AAGUGCCGCCAUCUUUUGAGUGU | SEQ ID NO: 5 |
| hsa-miR-4784 | UGAGGAGAUGCUGGGACUGA | SEQ ID NO: 6 |

### FIGURES:

- Figure 1A:: Human iPSCs generated from skin fibroblasts of healthy probands and differentiated into cardiomyocytes (human iPSC-derived cardiomyocytes, hiPSC-CMs). Immunofluorescence images showing Hoechst and cardiac troponin T positive hiPSC-CMs. Scale bar: 50 µm.
- Figure 1B:: Representative images of mock-transfected (upper image) and fluorescently labeled pre-miRNA (FAM-miR) transfected hiPSC-CMs at 24 hours post transfection (30nM, middle; 60 nM, lower image). Scale bar: 50 µm.
- Figure 1C:: Human iPSC-CMs transfected with fluorescently labeled FAM-miR were analyzed using fluorescence-activated cell sorting (FACS) 24 hours after transfection. Shown are representative flow cytometry plots of human iPSC-CM without (left plot) and with FAM-miR (right plot).
- Figure 1D:: Transfection efficiency as analyzed by FACS analysis (n=3) ; ***; P < 0.001 vs. Control.
- Figure 1E:: Representative images of human iPSC-CMs after mock-transfection (top) and transfection with cell death-inducing siRNAs (bottom) showing a high induction of a cell-death phenotype at 48 hours post transfection. Scale bar: 50 µm.
- Figure 2A:: MiRNA-expression in hiPSC-CMs after transfection with miR-mimic-1825. (n=3); ***; P < 0.001 vs. Mock.
- Figure 2B: MiRNA-expression in hiPSC-CMs after transfection with anti-miR-195. (n=3); ***; P < 0.001 vs. Mock.
- Figure 2C:: Incorporation of EdU in hiPSC-CMs treated with transfection reagent (Mock) and after transfection with miRNA-scrambled, miR-mimic-1825 (30nM) and miR-mimic-1825 (60nM). (n=3); **; P < 0.01 vs. Mock) or miR-scrambled.
- Figure 2D:: Representative immunofluorescence images of EdU-uptake in mock transfected hiPSC-CMs, and after transfection with miRNA-scrambled, miR-mimic-1825 (30nM) and miR- mimic-1825 (60nM). Scale bar: 50 µm.
- Figure 2E:: Lactate dehydrogenase (LDH) release of hiPSC-CMs after transfection with miR-scrambled and miR-mimic-1825 (60nM). Supernatants were collected 24 hours post transfection. (n=3).
- Figure 2F:: Incorporation of EdU in hiPSC-CMs transfected with miR-scrambled or miR-mimic-1825 under normoxia or after transient hypoxia for two hours and four hours. EdU-positive hiPSC-CMs were assessed 96 hours post transfection. *, miR-scrambled vs. miR-mimic-1825; #, miR-mimic-1825 normoxia vs hypoxia (n=3). *; #; P < 0.05, **; P < 0.01; ***; P < 0.001.
- Figure 3A:: Graphical illustration of the high-throughput (HT) screening workflow. A miRNA-library was used for overexpression (miR-mimics) and inhibition (anti-miRs) of 2019 miRNAs. HT screen was performed in duplicates.
- Figure 3B:: Immunofluorescence images of hiPSC-CMs showing Hoechst(blue, nuclei), cardiac troponin T (green) and EdU (red) after transfection with miR-mimic-519e-3p, miR-mimic-515-3p and mock transfection. Scale bar: 50 µm.
- Figure 3C:: EdU-incorporation into hiPSC-CMs was assessed after individual transfection with 2019 miRNA-inhibitors (anti-miRNAs). Data plot indicates EdU-incorporation (%). A Z-factor > 3 in both replicates, indicated by red dots, was used to delineate a significant increase in EdU-uptake.
- Figure 3D:: Two anti-miRNAs, let-7c-5p and miR-365-3p significantly increased EdU-uptake in hiPSC-CM. The horizontal dotted line indicates the mean EdU-incorporation of hiPSC-CMs.
- Figure 3E:: Validation of anti-miRNAs let-7c-5p and miR-365-3p in a second screen. However, transfection of hiPSC-CM with anti-let-7c-5p and anti-miR-365-3p did not significantly increase EdU-uptake as compared to mock transfected hiPSC-CMs.
- Figure 3F:: EdU-incorporation into hiPSC-CMs was assessed after individual transfection with 2019 miRNA-mimics. Data plot indicates EdU-incorporation (%). A Z-factor > 3 in both replicates, indicated by red dots, was used to delineate a significant increase in EdU-uptake.
- Figure 3G:: Twenty eight miRNA-mimics significantly increased EdU-uptake in hiPSC-CM. The horizontal dotted line indicates the mean EdU-incorporation of hiPSC-CMs. Light colored bars highlight members of the miR-515-family and members of the miR-371-373 Cluster.
- Figure 3H:: Validation of miRNA-mimics in a second screen. Screen was performed in duplicates, shown is the mean EdU-uptake of two screens.
- Figure 4A:: Incorporation of EdU in hiPSC-CMs transfected with miR-scrambled, miR-mimic-515-3p, miR-mimic-519e-3p and miR-mimic-371a-3p under normoxia. (n=3). ***; P < 0.001 vs. miR-scrambled.
- Figure 4B:: Incorporation of EdU in hiPSC-CMs transfected with miR-scrambled, miR-mimic-515-3p, miR-mimic-519e-3p and miR-mimic-371a-3p after transient hypoxia. (n=3). *, miR-scrambled vs. miR-mimic-515, 519 & 371; $, miR-515 vs. 371; #, miR-519 vs. miR-371. #; P < 0.05, $$; P < 0.01, ***; P < 0.001.
- Figure 4C:: EdU-uptake significantly increases after transient hypoxia in miR-mimic-515-3p, miR-mimic-519e-3p transfected hiPSC-CMs, but not after miR-scrambled or miR-mimic-371a-3p transfection. *, normoxia vs. hypoxia. (n=3). *; P < 0.05, **; P < 0.01.
- Figure 4D:: Representative immunofluorescence images of hiPSC-CMs after transfection with miR-mimic-515-3p, miR-mimic-519e-3p and miR-mimic-371a-3p, followed by transient hypoxia. Hoechst (blue), cardiac troponin T (red) and EdU (green). Scale bar: 50 µm.
- Figure 5A:: Analysis of mitosis in hiPSC-CM, as assessed using phosphorylated histone H3 (H3P). H3P-positive hiPSC-CM after transfection with miR-scrambled, miR-mimic-515-3p, miR-mimic-519e-3p and miR-mimic-371a-3p under normoxia. (n=5). *; P < 0.05, **; P < 0.01; ***; P < 0.001 vs. miR-scrambled.
- Figure 5B:: H3P-positive hiPSC-CM after transfection with miR-scrambled, miR-mimic-515-3p, miR-mimic-519e-3p and miR-mimic-371a-3p after transient hypoxia. (n=5). *; P < 0.05, ***; P < 0.001 vs. miR-scrambled.
- Figure 5C:: Transient hypoxia in miR-mimic-515-3p, miR-mimic-519e-3p transfected hiPSC-CMs, but not after miR-scrambled or miR-mimic-371a-3p transfection, substantially increase H3P-positive hiPSC-CM. (n=5). *, normoxia vs. hypoxia. ***; P < 0.001.
- Figure 5D:: Representative immunofluorescence images of hiPSC-CMs after transfection with miR-scrambled, miR-mimic-515-3p, miR-mimic-519e-3p and miR-mimic-371a-3p, followed by transient hypoxia. Hoechst (blue), cardiac troponin T (green) and H3P (red). (n=5). Scale bar: 50 µm.
- Figure 6A:: Incorporation of EdU in hiPSC-CMs after transfection with increasing miRNA-mimic concentrations (20, 40, 60, 80 and 100 nM). $, miR-scrambled (20nM-100nM) vs. miR-515 and miR 519 (20nM-100nM). *, vs. miR-515 in between different miRNA-concentrations (20nM-100nM). #, vs. miR-519 in between different miRNA-concentrations (20nM-100nM). (n=3). $; P < 0.05, **; $$; P < 0.01; ***; $$$; ### P < 0.001.
- Figure 6B:: Representative immunofluorescence images of hiPSC-CMs showing Hoechst (blue, nuclei), cardiac troponin T (red) and EdU (green). Scale bar: 50 µm.
- Figure 6C:: Analysis of cytotoxicity, as assessed by LDH release of hiPSC-CMs after transfection with miR-mimic-515-3p, miR-mimic-519e-3p and miR-mimic-590 using different miRNA-mimic-concentrations. (n=3). *, treatments within miRNA-scrambled (20 nM vs. 40nM; 40nM vs. 60nM; 60nM vs. 100 nM). ***; P < 0.001.
- Figure 6D:: EdU-uptake, as assessed at day 3 to day 6 post transfection, in hiPSC-CMs treated with miR-scrambled, miR-mimic-515-3p, miR-mimic-519e-3p and miR-mimic-590 from day three to six after transfection. (n=3). *, vs. miR- scrambled; $, miR-515 from day 3 to 6; #, miR-519 from day 3 to 6. *,$,#, P < 0.05, **; $$,##, P < 0.01; ***,$$$, ###, P < 0.001
- Figure 6E:: H3P-positive hiPSC-CMs, assessed at day 2 to day 5 post transfection, after treatment with miR-mimic-515-3p, miR-mimic-519e-3p and miR-mimic-590. (n=3). *, vs. miR- scrambled; *; P < 0.05, ***; P < 0.001.
- Figure 6F:: Incorporation of EdU does not significantly differ in miRNA-mimic-transfected hiPSC-CMs from female as compared to male donors. (n=3). *, vs. miR- scrambled (female hiPSC-CMs). *; P < 0.05, **; P < 0.01. $, vs. miR-scrambled (male hiPSC-CMs). $$; P < 0.01; $$$;P < 0.001.
- Figure 6G:: H3P-positive hiPSC-CMs transfected with miR-mimics in female as compared to male donors. (n=3). *, vs. miR-scrambled (female hiPSC-CMs). *; P < 0.05, **; P < 0.01. $, vs miR-scrambled (male hiPSC-CMs). $$; P < 0.01.
- Figures 7A-C:: Quantitative real-time PCR (qPCR) of cell- cycle inhibitors (CDKN1A, CDKN1B, and CDKN1C) in hiPSC-CMs after transfection with miR-scrambled, miR-mimic-515-3p or miR- mimic-519e-3p. (n=4-5). *, vs. miR-scrambled. ***; P < 0.001.
- Figures 7D-F:: Quantitative real-time PCR (qPCR) of cell-cycle activators (CCNA2, CCNB1, and CCND2) in hiPSC-CMs after transfection with miR-scrambled, miR-mimic-515-3p or miR- mimic-519e-3p. (n=4-5). *, vs. miR-scrambled. **; P < 0.01; ***; P < 0.001.
- Figures 7G-H:: Quantitative real-time PCR (qPCR) of Hippo pathway activators (LATS1 and MOB1B) in hiPSC-CMs after transfection with miR-scrambled, miR-mimic-515-3p or miR- mimic-519e-3p. (n=4-5). *, vs. miR-scrambled. *; P < 0.05, **; P < 0.01; ***; P < 0.001.
- Figure 7I:: Quantitative real-time PCR (qPCR) of cytokinesis marker Aurora B in hiPSC-CMs after transfection with miR-scrambled, miR-mimic-515-3p or miR- mimic-519e-3p. *, vs. miR-scrambled. (n=4-5). **; P < 0.01; ***; P < 0.001.
- Figures 7J-M:: Quantitative real-time PCR (qPCR) of sarcomeric genes involved in maturation/differentiation (MYL2, MYL7, MYOM2, and MYOM3) in hiPSC-CMs after transfection with miR-scrambled, miR-mimic-515-3p or miR- mimic-519e-3p. (n=4-5). *, vs. miR-scrambled. *; P < 0.05, ***; P < 0.001.
- Figures 7N-O:: Quantitative real-time PCR (qPCR) of genes involved in cardiomyocyte hypertrophy in hiPSC-CMs after transfection with miR-scrambled, miR-mimic-515-3p or miR- mimic-519e-3p. (n=4-5). *, vs. miR-scrambled. ***; P < 0.001.
- Figure 8A:: Western blot analysis from hiPSC-CMs after transfection with miR-scrambled, miR-mimic-515-3p or miR-mimic-519e-3p using indicated antibodies.
- Figure 8B:: Cell-cycle inhibitor CDKN1A is significantly reduced after miR-mimic-515-3p or miR-mimic-519e-3p treatment. (n=3-4). *, vs. miR-scrambled. **; P < 0.01; ***; P < 0.001
- Figure 8C:: Cell-cycle activator CCNA2 is substantially increased after miR-mimic-515-3p or miR-mimic-519e-3p treatment. (n=3-4). *, vs. miR-scrambled. **; P < 0.01; ***; P < 0.001.
- Figure 8D:: Cytokinesis marker Aurora B show a substantially increased expression after miR-mimic-515-3p or miR-mimic-519e-3p treatment. (n=3-4). *, vs. miR-scrambled. ***; P < 0.001
- Figure 8E:: Representative immunofluorescence images of Aurora B (red) in miR-scrambled, miR-mimic-515-3p or miR-mimic-519e-3p- transfected hiPSC-CMs. Arrows pointing to hiPSC-CMs undergoing cytokinesis. Scale bar: 50 µm. Outlined regions are magnified in the bottom panels. Scale bar: 50 µm.
- Figure 9A:: Incorporation of EdU in mouse cardiomyocytes after transfection with hsa-miR-mimic-515-3p and hsa-miR-mimic-519e-3p. (n=3). *, vs. miR- scrambled. *; P < 0.05, **; P < 0.01; ***; P < 0.001.
- Figure 9B:: Representative immunofluorescence images of mouse cardiomyocytes showing Hoechst (nuclei), cardiac troponin T (red) and EdU (green). Scale bar: 50 µm.
- Figure 9C:: H3P-postive cells in mouse cardiomyocytes after transfection with hsa-miR-mimic-515-3p and hsa-miR-mimic-519e-3p, *, vs. miR-scrambled.
- Figure 9D:: As in Figure 9B. Arrows pointing to H3P-positive mouse cardiomyocytes. (n=3). ***; P < 0.001.
- Figure 10A:: The Venn diagram shows overlapping and differentially expressed genes in hiPSC-CM after miR-mimic-519e-3p and miR-mimic-515-3p treatment as compared to miR-scrambled transfected hiPSC-CMs.
- Figure 10B:: Volcano plot of differentially expressed genes after transfection with miR-519e-3p as compared to miR-scrambled transfected hiPSC-CMs. Green and red circles indicate statistically significant differentially expressed genes with fold change > 1.5 and p-value 0.05. Abscissa: Fold-change; vertical coordinates: p-value (n=4 per group).
- Figure 10C:: Heat map, arranged by hierarchical clustering, of differentially expressed genes (miR-519e-3p (right rows) vs. miR-scrambled (left rows)). The color represents the relative expression level (log2 scaled FPKM). Red, increased expression; green: reduced expression
- Figure 10D:: Top ten enriched gene ontology (GO) terms of downregulated genes, ordered from top to bottom by p-value.
- Figure 10E:: Top ten enriched gene ontology (GO) terms of upregulated genes, ordered from top to bottom by p-value.
- Figure 10F:: Cord plots showing top nine GO biological process terms that are related to the top 20 significantly differentially downregulated genes.
- Figure 10G:: Cord plots showing top nine GO biological process terms that are related to the top 20 significant differentially upregulated genes.
- Figures 11:: Overexpression of hsa-miR-515-3p and hsa-miR-519e-3p induce cytokinesis with sarcomeric disassembly in hiPSC-CM.
- Figure 11A:: Representative immunofluorescence images of Aurora B-kinase localization in different phases of mitosis in hiPSC-CMs after transfection with miR-mimic- 519e-3p. Dapi (blue, nuclei), cardiac troponin T (green) and Aurora B-kinase (red) positive hiPSC-CMs were assessed 48 hours post transfection. Scale bar = 50 µm.
- Figure 11B:: Representative immunofluorescence images for illustration of sarcomeric disassembly in hiPSC-CMs after transfection with miR-mimic-515-3p and miR-mimic-519e-3p. Hoechst (blue, nuclei), sarcomeric alpha-Actinin (green). Scale bar = 50 µm.
- Figure 11C:: Analysis of Aurora B-kinase-positive hiPSC-CM after transfection with miR-scrambled, miR-mimic-515-3p and miR-mimic-519e-3p. (n=3). **P<0.01; ***P<0.001 vs. miR-scrambled.
- Figure 11D:: Analysis of midbodies as assessed with Aurora B-kinase after transfection with miR-scrambled, miR-mimic-515-3p and miR-mimic-519e-3p. (n=3). ***P<0.001 vs. miR-scrambled.
- Figure 11E:: Representative immunofluorescence images of hiPSC-CMs after transfection with miR-scrambled, miR-mimic-515-3p and miR-mimic-519e-3p for quantification of Aurora B-kinase. Arrows pointing to hiPSC-CMs undergoing cytokinesis. Magnification of insets below. Dapi (blue, nuclei), cardiac troponin T (green) and Aurora B-kinase (red). (n= 3) Scale bar = 50 µm.
- Figures 12:: RNA-Seq reveals a substantial modulation of biological processes involved in cardiomyocyte proliferation after miR-mimic-515-3p and miR-mimic-519e-3p overexpression in hiPSC-CM. HiPSC-CMs were treated with scrambled-miRNA (Scrambled), miR-mimic-515-3p (miR-515) and miR-mimic-519e-3p (miR-519) (n=4 per group). RNA was isolated and subjected to RNA-seq.
- Figure 12A:: Volcano plots show overlapping and differentially expressed genes in hiPSC-CM after miR-mimic-515-3p and miR-mimic-519e-3p treatment as compared to miR-scrambled transfected hiPSC-CMs.
- Figure 12B:: Euler diagram show overlapping and differentially expressed genes in hiPSC-CM after miR-mimic-515-3p and miR-mimic-519e-3p treatment as compared to miR-scrambled transfected hiPSC-CMs.
- Figure 12C:: Heat map, arranged by hierarchical clustering of differentially expressed genes (miR-mimic-515-3p (left rows), miR-mimic-519e-3p (middle rows), miR-scrambled (right rows). The color represents the relative expression level. Red, increased expression; blue: reduced expression.
- Figure 12D:: Two clusters were identified after miR-mimic-515-3p and miR-mimic-519e-3p as compared to miR-scrambled transfection in hiPSC-CM. Enriched gene ontology (GO) terms of downregulated (left) and upregulated (right) genes.
- Figure 12E:: Shown are differentially expressed genes (false discovery rate (FDR) <0.05) of PI3K-Akt signaling pathway involved in cardiomyocyte proliferation. P-Value for representative pathway is shown on lower right of the heat maps.
- Figure 12F:: Shown are differentially expressed genes (false discovery rate (FDR) <0.05) of WNT/B-catenin signaling pathway involved in cardiomyocyte proliferation. P-Value for representative pathway is shown on lower right of the heat maps.
- Figure 12G:: Shown are differentially expressed genes (false discovery rate (FDR) <0.05) of TGF-B/SMAD signaling pathway involved in cardiomyocyte proliferation. P-Value for representative pathway is shown on lower right of the heat maps.
- Figure 12H:: Shown are differentially expressed genes (false discovery rate (FDR) <0.05) of Notch signaling pathway involved in cardiomyocyte proliferation. P-Value for representative pathway is shown on lower right of the heat maps.
- Figure 12I:: Shown are differentially expressed genes (false discovery rate (FDR) <0.05) of Hippo/YAP signaling pathway involved in cardiomyocyte proliferation. P-Value for representative pathway is shown on lower right of the heat maps.
- Figure 12J:: Shown are differentially expressed genes (false discovery rate (FDR) <0.05) of NRG/ERBB signaling pathway involved in cardiomyocyte proliferation. P-Value for representative pathway is shown on lower right of the heat maps.
- Figure 12K:: Analysis of transcription factors related to cardiomyocyte proliferation that are significantly upregulated after miR-mimic-transfection in hiPSC-CMs.
- Figure 12L:: Gene ontology analysis for differentially expressed genes (false discovery rate (FDR) <0.05) in response to hypoxia after miR-mimic-transfection in hiPSC-CMs. P-Value for representative pathway is shown on lower right of the heat maps.
- Figures 13:: Intramyocardial delivery of hsa-miR-mimic-519e-3p post myocardial infarction induces cardiomyocyte proliferation in adult mouse. Data was assessed three days post LAD ligation and intramyocardial injection.
- Figure 13A:: Graphical illustration of the workflow. Three days post myocardial infarction, hearts were harvested and further processed for immunohistochemistry to detect proliferating cardiomyocytes.
- Figure 13B:: EdU-positive cardiomyocytes after treatment with hsa-miR-mimic-519e-3p or miR-scrambled (n=4). ***P<0.001, vs. miR-scrambled.
- Figure 13C:: H3P-positive cardiomyocytes after treatment with hsa-miR-mimic-519e-3p or miR-scrambled (n=4). (n=4). *; P < 0.05, vs. miR-scrambled.

### Key Resource Table:

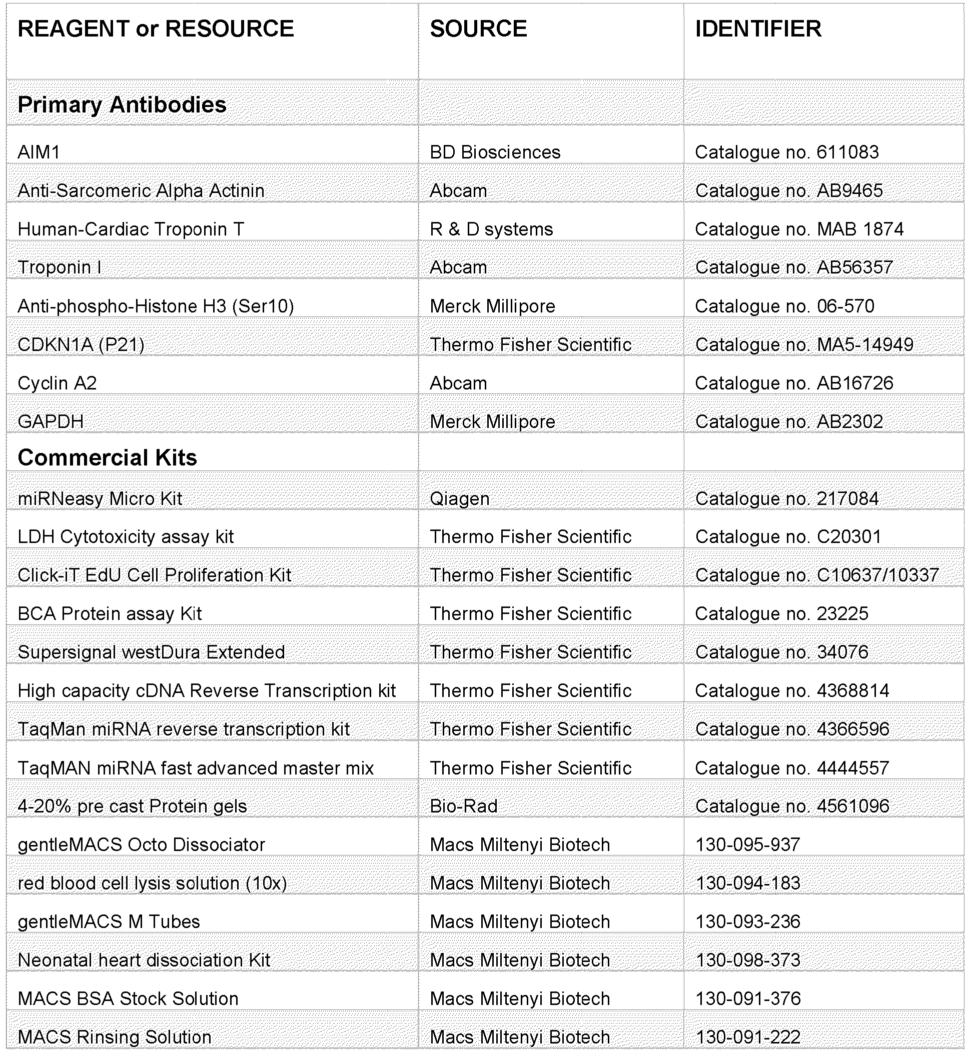

### Methods:

### Generation of hiPSC-derived cardiomyocytes

As model system, human induced pluripotent stem cell (hiPSC)-derived cardiomyocytes (CMs) (hiPSC-CM) were used. Female and male donors were used to generate cardiomyocytes, with two different reprogramming based approaches. Two female hiPSC lines were generated with a episomal plasmid based transduction. One female and one male hiPSC line was reprogrammed with the STEMCCA system, which is a humanized excisable lentiviral system containing all four reprogramming factors OCT4, SOX2, KLF4, and c-MYC in a single "stem cell cassette"(pHAGE2-EF1aFull-hOct4-F2A-hKlf4-IRES-hSox2-P2A-hcMyc-W-loxP). The pluripotent clones were expanded up to passage 8. The established iPSCs were proven for their pluripotency by both in vitro studies - the expression of pluripotency markers and differentiation experiments - and in vivo teratoma formation assay.

### Cardiomyocyte selection and culture

Post differentiation, cardiac culture medium (RPME 1640 with Glutamax and HEPES, B27 supplement) was aspirated and replaced with cardio selection medium (RPMI 1640 without Glucose, Lactate/HEPES, hAlbumin, human recombinant 100mg L-Ascorbic Acid 2-Phosphate). The medium was exchanged every second day. Post selection, the medium was replaced with cardio culture medium. The iPSCs were effectively differentiated and selected in vitro into > 95% pure cardiomyocytes by using the established technologies. Human iPSC-derived cardiomyocytes were used for experiments after 30-40 days post selection. Cell detachment was performed using 0.25% trypsin, 0.02% EDTA and Stempro accutase, followed by collagenase diluted in RPME 1640 for cell dissociation. Thereafter, cells were seeded in cardio digestion medium (RPME 1640 with Glutamax and HEPES, B27 Supplement, 10% FBS, Thiazovivin (2µM)). Medium was exchanged to cardio culture medium 48 hours later. Cell culture plates were coated with Geltrex for human iPSC-CM and fibronectin for neonatal mouse cardiomyocytes.

### MiRNA and siRNA transfection of human iPSC-CM:

Lullaby stem (OZ Biosciences) was used for transfection of microRNAs using a standard forward transfection protocol. Medium was replaced with fresh cardio culture medium 24 hours post transfection. 48 hours post transfection, hiPSC-CM were exposed to hypoxia (0.5% oxygen, 5% CO2) for 2 hours. 50 hours post transfection, medium was exchanged to cardio culture medium containing EdU, or hiPSC-CM were fixed and stained with H3P or Aurora B and further proceeded for immunocytochemistry. Assessment of FAM-miR transfected hiPSC-CM was performed 24 hours post transfection using fluorescent imaging or fluorescence activated cell sorting (FACS). For FACS analysis, cells were briefly washed in PBS, trypsinized, and the detached cells were resuspended with freshly mixed staining solution (100 µl Annexin-biding buffer + 2uL Annexin V-Pacific Blue + 1 uL Propidium iodide for each sample). The mix was carefully vortexed and added to annexin binding buffer and measured immediately at an attune flow cytometer. All stars siRNA-cell death control (Qiagen) was used to observe a cell death phenotype in hiPSC-CM 48 hours post transfection.

### Isolation and culture of neonatal cardiomyocytes from a mouse heart:

The experiments have been performed under license number TS-9/2016 Nephropatho. "The investigation conforms with the guidelines from Directive 2010/63/EU of the European Parliament on the protection of animals used for scientific purposes. Extraction of organs and preparation of primary cell cultures were approved by the local Animal Ethics Committee in accordance to governmental and international guidelines on animal experimentation (protocol TS-9/2016 Nephropatho).

Mice (P3) were decapitated at postnatal day 3, the chest was opened, and hearts were removed with a curved forceps. Atria was removed and ventricles were minced as small as possible using a scalpel blade. For the isolation of cardiomyocytes, minced hearts were digested using gentleMACS Octo Dissociator (Miltenyi Biotech) according to the manufacturers instruction. For cardiomyocyte enrichment, cells were incubated with pre-plating medium (DMEM/F-12 +GlutaMAX; supplemented with: 10% [v/v] FBS 1% [v/v] Pen/Strep 2 mM L-Glutamine). After 90 minutes, the non-attached, enriched P3 cardiomyocytes, were collected in 50 ml falcon tubes. After centrifugation, cells were resuspended in neonatal culture medium (DMEM/F-12 Glutamax medium supplemented with, 1% Penicillin and Streptomycin and 5% FBS, 2% Horse serum (HS)), counted using hemocytometer and plated with appropriate density. 24 hours later, the medium was replaced with fresh neonatal culture medium and cells were used for transfections.

### MiRNA transfection of mouse cardiomyocytes:

For transfection experiments, 96 well plates were pre coated with fibronectin. Lipofectamine RNAiMAX, was used to transfect P3 mouse cardiomyocytes, according to the manufacturer protocols. The transfection reagent was mixed together with microRNA and reduced serum medium (Opti-MEM) and mixture was slowly added dropwise on to the cells. Medium exchange was performed 24 hours post transfection followed by incubation with EdU or stained with H3P.

### Functional high-throughput screening

A high-throughput (HT) screening by using a miRNA-library (Ambion; mirVana Human Library v19.0; 2019 miR-mimics and miR-inhibitors) was performed in two parallel screenings. For the HT screen, a forward transfection protocol was used. Human iPSC-CMs were plated into Geltrex coated 384-well plates (BD Falcon). Each individual miRNA of the library (miR-mimic or miR-inhibitor, 60nM) was robotically transferred using a Freedom EVO liquid handling workstation (Tecan) after complexing with Lullaby Stem (OZ Biosciences) according to the manufacturer protocol. The mixture was slowly added drop by drop. MiRNA transfected hiPSC-CMs were kept for 24 hours at 37 °C in 5% CO2. Medium exchange was performed by a robotic dispenser after 24 hours. 48 hours post transfection, hiPSC-CMs were exposed to a hypoxia (0.5% oxygen, 5% CO2) for 2 hours. Thereafter, cells were replaced with cardio culture medium containing EdU and incubated for 48 hours. Three days post transfection, cells were fixed and processed for immunofluorescence analysis.

### High content imaging, image analysis and data processing of human iPSC-CMs

HiPSC-CMs were fixed and stained. The immunofluorescence imaging was performed by ArrayScan XTI High Content Screening Platform (ThermoScientific). 20X magnification, with three imaging channels were used. Channel 1 was represented as Hoechst (Nucleus marker), Channel 2 as Troponin T (Cardiac specific Marker) and Channel 3 for EdU or H3P. 36 individual images from each channel were obtained from a single well of a 384 well plate. Images were analyzed using cellomics scan Version 6.6.0 (ThermoScientific HCS Studio). Once the required imaging analysis was obtained, the data was processed using the KNIME analytics platform (KNIME). Z-score is represented as standard deviations from being above or below the mean of data points, negative or below a Z-score represents that the acquired data is below the mean average and positive or above a Z-score represents that the acquired data is above the mean average of the data.

### RNA extraction from hiPSC-CM

Total RNA was isolated from hiPSC-CMs using the RNeasy Kit (Qiagen). Kits were used according to the manufacturer instructions. HiPSC-CM were seeded and transfected in 24- or 48-well cell culture plates. 48 hours and 72 hours post microRNA-transfection, cell culture medium was aspirated and cells were washed three times with sterile DPBS. DPBS was aspirated, later QlAzol lysis reagent (350 to 700 µl) was added onto cells and incubated for 5 minutes at RT. The resulting cell lysates were collected in 1.5 ml nuclease free reaction tubes (Eppendorf). MiRNeasy RNA extraction kit (Qiagen) was used to isolate the RNA according to the manufacturer instructions. The RNA concentration was measured using Nano drop at 260/280 nm. The isolated RNA was dissolved in 14 µl of RNAse-free water and stored at -80 °C or continued for reverse transcription.

### Reverse transcription (cDNA synthesis) and quantitative real-time PCR (qRT-PCR)

qRT-PCR was performed according to standard protocols. The isolated RNA was dissolved in 14 µl of RNAse-free water and stored at -80 °C or continued for reverse transcription. High-capacity cDNA reverse transcription kit (Thermo fisher scientific) was used. Syber green detection method was used for performing qRT-PCR. qRT-PCR was performed in 384 well plate and analyzed by Viia R real time PCR system (Applied Bio systems). Changes in gene expression were analyzed by relative quantification of mean Ct value of a housekeeping gene (GAPDH) which served as a normalizing control. The relative expression was calculated by 2-ΔΔCT formula. For expression of miRNA, RNA was isolated after transfection of hiPSC-CMs with hsa-miR-mimic-1825 and hsa-anti-miR-195-5p (Thermo fisher scientific) as mentioned above. TaqMan microRNA reverse transcription kit was used to perform reverse transcription and TaqMan microRNA fast advanced master mix and TaqMan microRNA primers were used. Expression of miR-1825 and miR-195-5p was normalized with that of RNU48.

### RNA-sequencing

MiRNA-scrambled, hsa-miR-mimic-515-3p and hsa-miR-mimic-519e-3p were transfected into human iPSC-CM as described above. 72 hours post transfections, cells were washed with DPBS three times. Thereafter QlAzol (Qiagen- MiRNeasy RNA extraction) was added. Resulting lysates were collected in 1.5 ml nuclease free reaction tubes (Eppendorf). RNA-Sequencing and analysis was performed by ArrayStar (Arraystar, Inc., USA). Total RNA was used to prepare the sequencing library using KAPA Stranded RNA-Seq Library Prep Kit (Illumina). Libraries were sequenced using HiSeq 4000 (Illumina). Image analysis and base calling was performed using the Solexa pipeline v1.8 (OffLine Base Caller software, v1.8). The trimmed reads (trimmed 5',3'-adaptor bases using cutadapt) were aligned to reference genome using Hisat2 software (v2.0.4). The transcript abundances for each sample was estimated with StringTie (v1.2.3), and the FPKM value for gene and transcript level were calculated with R package Ballgown (v2.6.0). Hierarchical clustering, gene ontology, pathway analysis and volcano plots were performed with the differentially expressed genes in R, Python or shell environment for statistical computing and graphics.

### Western blotting

HiPSC-CMs were transfected with individual microRNAs. 72 hours post transfections, cells were incubated with freshly prepared RIPA lysis buffer. Cell lysates were collected and centrifuged at 13000 rpm for 20 minutes at 4°C. Supernatants from centrifugation were collected and the pellet was discarded. BCA protein assay kit (Pierce) was used to determine the protein concentration. Equal amount of proteins were resolved by 4-20% Pre cast protein gels from (Bio-Rad) and blotted onto a polyvinylidene difluoride (PVDF) membrane. Membranes were blocked with 5% Milk or 5% BSA, incubated with primary antibodies over night at 4°C, followed by incubation with HRP-linked secondary antibodies. Chemiluminescent substrate (Supersignal westDura Extended) solution was added onto the membrane and the membrane was developed under chemiluminesence to detect antibody-labeled proteins (INTAS Detection System).

### Lactate dehydrogenase (LDH) Cytotoxicity assay

LDH assay was performed with the LDH assay kit (Pierce, Thermo scientific) according to the manufacturer protocols. HiPSC-CMs were plated in 96 or 384 well plates, and transfected with either scrambled-miRNA or miRNA-mimics using concentrations ranging from 20nM to 100 nM to assess toxicity. 24 hours post transfection, the medium was carefully collected and placed in a 384 well plate. LDH start reagent was added onto the cells and incubated for a period of 30 minutes. Thereafter, LDH assay stop solution was added to stop the reaction and measured with the Tecan florescent plate reader at 490nm and 690nm absorbance.

### Statistical analysis

Data are presented as ± standard error of the mean (SEM). Bars represent average of a minimum of three to five experiments. Data analysis was conducted using Graphpad prism 7 software. We analyzed the data using un-paired Student's t testOne way or Two-way ANOVA for multiple comparisons, using Tukey's or Sidak's post hoc tests. Error bars represent mean ± s.e.m. *,#.$ P ≤ 0.05, **, ##, $$ P ≤ 0.01, ***, ###, $$$ P ≤ 0.001. A value of P ≤ 0.05 was considered statistically significant.

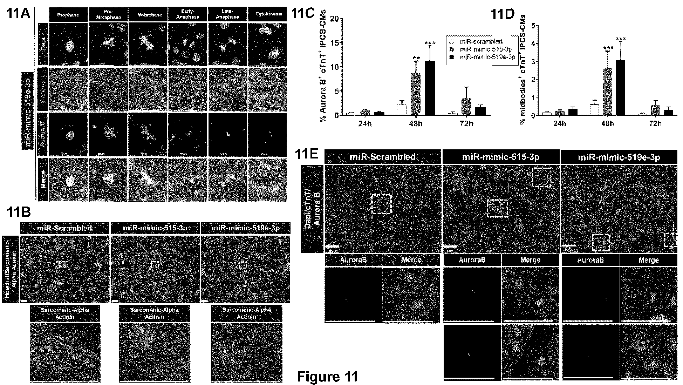

The following methods are relevant in particular for the results presented in Fig. 11, 12 and 13:

### Immunocytochemistry

Post miR-mimic transfection, hiPSC-CM were washed shortly with PBS. For staining, hiPSC-CM were fixed with 3.7% formaldehyde (Sigma). Cells were washed three times with PBS and permeabilized with 0.5 % Triton X-100 in PBS, and washed three times in PBS. HiPSC-CM were blocked for 1 hour at RT with blocking buffer (10% Horse serum, 1 % BSA, 0.3 % Triton) in PBS and incubated with primary antibody Aurora B Kinase (BD Transduction laboratories) in dilution buffer (1x PBS, 1% BSA, 1% Donkey or horse serum, 0, 3% Triton) overnight at 4 °C. Subsequently, hiPSC-CM were washed three times with 0.1 % Nonidet P40 in PBS, and incubated with corresponding secondary antibodies conjugated with Alexa Fluor 488 or Alexa Fluor 594 (1:500 Life technologies) for 1 hour at RT. HiPSC-CM were washed three times with 0.1% BSA in PBS, incubated with Dapi (Nuclear stain) and washed three times with 1xPBS. The imaging was performed in three random fields. Per replicate, > 300 hiPSC-CMs positive for cardiac TroponinT were analyzed (n=3).

### Myocardial Infarction

Adult C57BL/6J male mice (8-12 weeks of age) were anesthetized, intubated and ventilated on a rodent ventilator. After left thoracotomy, myocardial infarction (MI) was induced by permanent ligation of the left anterior descending coronary artery. Hsa-miR-519e-3p or miR-scrambled (miRVana, ThermoScientific) were injected intramyocardially in the peri-infarct zone at three sites using a lipid-based transfection. Twenty-four hours after MI, mice received EdU (200 µl intraperitoneal). Hearts were harvested three days after MI. Mouse experiments were approved by the research advisory committee and permitted by LAGeSo (Landesamt für Gesundheit und Soziales Berlin).

### Immunohistochemistry of heart sections

Mouse hearts were washed in PBS, transferred to 4% PFA and incubated overnight at 4 °C. The day after, hearts were washed with PBS and transferred into a paraffin embedding cassette. Mounted sections were deparaffinized. Following antigen retrieval, sections were cooled down at RT, blocked in blocking buffer and incubated overnight at 4°C with primary antibodies, rabbit anti-phospho-Histone H3 (Ser10) (1:200, Millipore) and anti-sarcomeric alpha Actinin (1:100, Abcam). Subsequently, samples were washed with PBS and incubated with corresponding secondary antibodies conjugated to Alexa Fluor 488 and Alexa Fluor 594 (Invitrogen) and Click it EdU kit cocktail. Analysis was performed in random areas of heart sections. In miR-519e-3p treated group and miR-scrambled treated group, in total >20,000 cardiomyocytes positive for sarcomeric alpha-actinin were analyzed.

### Statistical analysis for Fig. 11, 12 and 13:

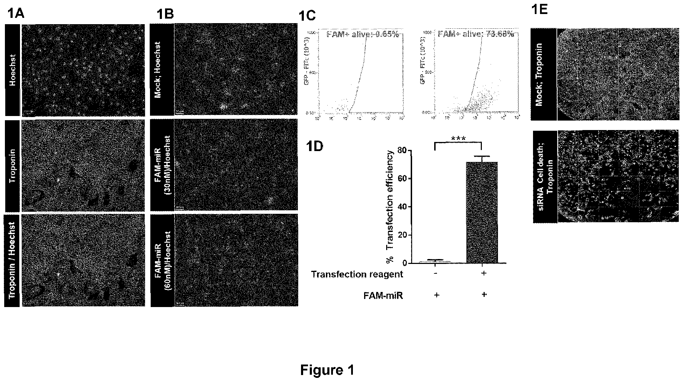

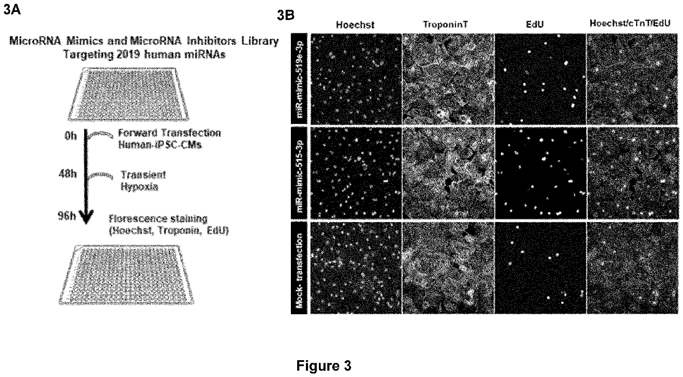

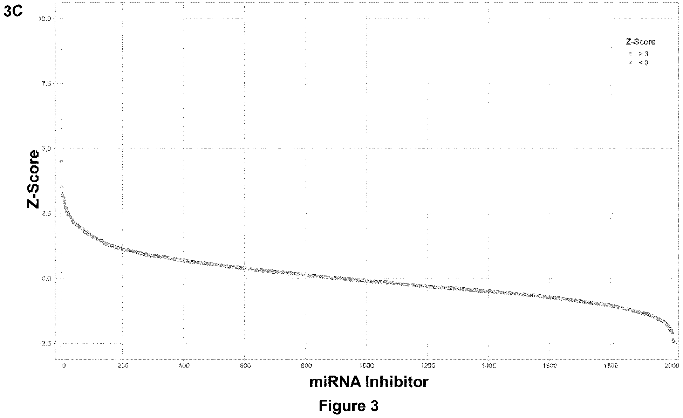

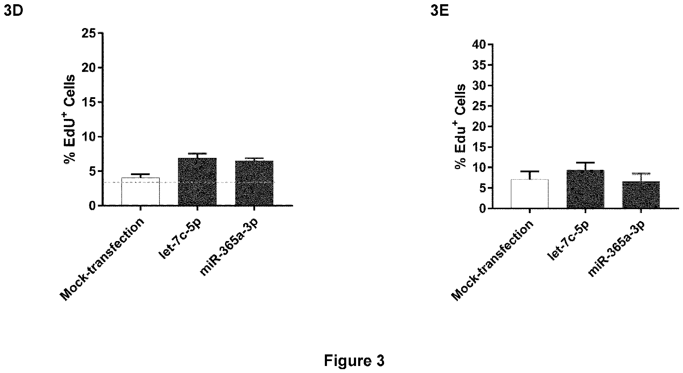

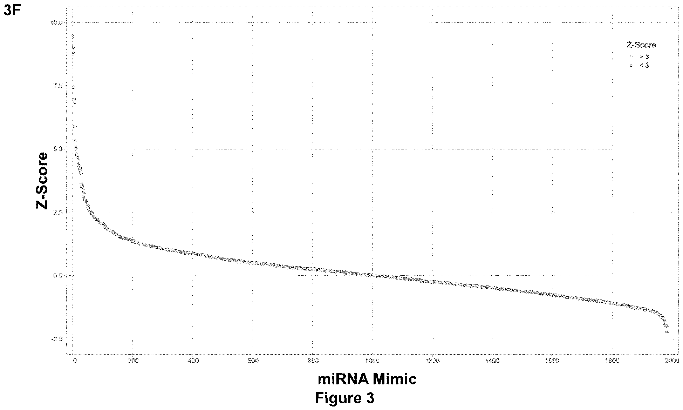

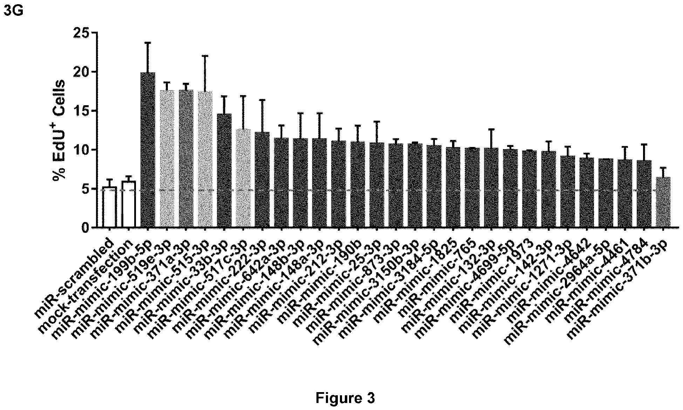

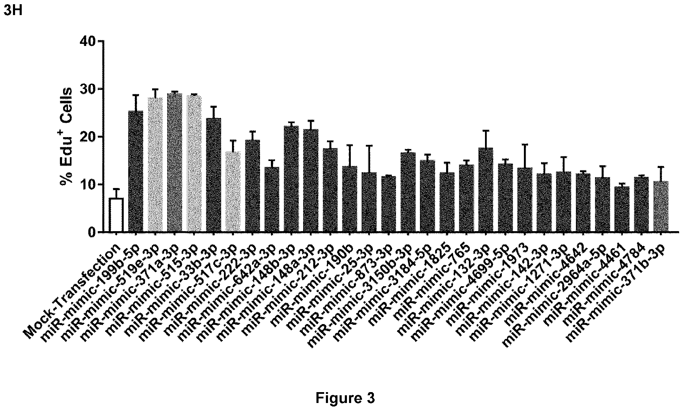

Data are presented as ± standard error of the mean (SEM). Bars represent average of a minimum of three to four experiments. Data analysis was conducted using Graphpad prism 7 software. We analyzed the data using Two-way ANOVA for (Figure 1), using Tukey's post hoc tests. Un-paired Student's t test for (Figure 3) and Error bars represent mean ± s.e.m. *, P ≤ 0.05, **, P ≤ 0.01, ***, P ≤ 0.001. A value of P ≤ 0.05 was considered statistically significant.

**Results:** A liposome-based transfection method was established for efficient delivery of miRNAs into hiPSC-CMs. The methodology and the results of evaluation are presented in Figure 1: As a model system, human cardiomyocytes (hiPSC-CM) that were differentiated from induced pluripotent stem cells (iPSC), were used and cultured for 30 to 40 days. A liposome-based transfection method was established to efficiently deliver miRNAs into hiPSC-CM (see Figure 1B-D). First, fluorescent-labelled miRNAs (FAM-miR) were transfected into hiPSC-CM (see Figure 1B) showing a high incorporation. FAM-microRNA transfected hiPSC-CMs further underwent FACS-analysis that confirmed efficient transfection (see Figure 1C-D). Furthermore, transfection with silencing RNAs (siRNAs) inducing cell death substantially initiated a cell-death phenotype at 48 hours (see Figure 1E).

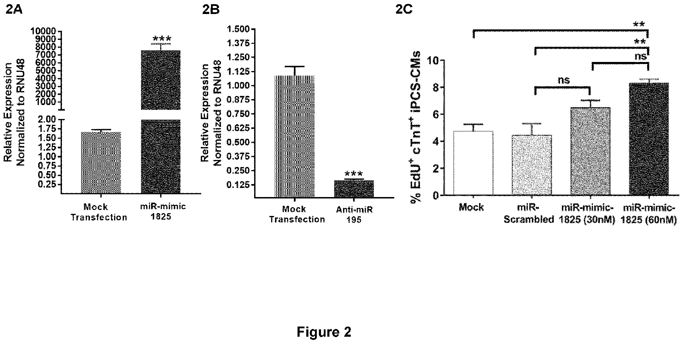

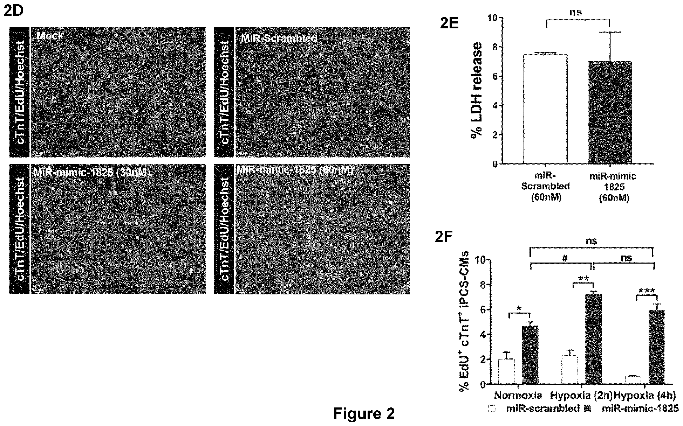

As shown in the Figures 2, hsa-miR-1825, a miRNA that is known to induce proliferation in murine cardiomyocytes, was used to test proliferative capacity in human iPSC-CMs. Transfection with miR-mimic-1825 increased incorporation of 5-Ethynyl-2-deoxyuridin (EdU) in a dose dependent manner (see Figures 2C, 2D). Furthermore, a protocol that mimics ischemia/reperfusion in hiPSC-CMs in vitro by exposing hiPSC-CMs to transient hypoxia increased cell-cycle reentry after miR-mimic-1825 transfection that was not observed after miR-scrambled transfection (see Figure 2F). Of note, transfection of miR-mimic-1825 did not enhance cytotoxicity in hiPSC-CMs, as assessed by LDH release (see Figure 2E). Furthermore, RT-qPCR showed significant increase of miR-1825 abundance after overexpression and downregulation of endogenous miR-195 after anti-miR-195 treatment (see Figures 2A, 2B), supporting efficient overexpression and downregulation of miRNAs using miRNA-mimics and anti-miRNAs, respectively.

A functional high-throughput screening was performed in human iPSC-CM with the library of miRNA-mimics and anti-miRNAs. The resulting proliferative activity of miRNA-transfected human iPSC-CMs was assessed by EdU and the results are shown in Figures 3:
The high-throughput screening was performed by using a miRNA-library consisting of 2019 miR-mimics and miR-inhibitors (anti-miRNAs) (Ambion; mirVana Human Library v19.0) in parallel screenings. For the high-throughput screening, each miRNA of the library (miR-mimic or anti-miRNA) was robotically transferred to a 384- well pre-plated with hiPSC-CMs. Individual forward transfection of miRNAs was performed with the established protocol and human iPSC-CMs were transiently exposed to hypoxia. Using a high-content imaging system, image segmentation was performed in order to detect proliferating cardiomyocytes by using Hoechst for nuclei staining.

Proliferation of miRNA-transfected human iPSC-CMs was assessed by EdU (see Figures 3A, 3B). Downregulation of 2019 miRNAs yielded only two miRNAs that increased proliferation (z-factor >3 in both duplicates), as assessed by EdU-positive cells (see Figures 3C and 3D). However, the increase of EdU-positive cardiomyocytes was modest (let-7c-5p: 6.9±0.3%, miR-365a-3p: 6.5±0.1%) (Figure 3D) and a cherry- pick screening did not confirm a significant increase in cell-cycling iPSC-CMs after treatment with miR-inhibitors, anti-let-7c-5p and anti-miR-365a-3p (Figure 3E) Conversely, transfection of miRNA-mimics identified twenty-eight miRNAs that increase proliferative activity of human iPSC-CMs (z-factor > 3 in both duplicates) (Figure 3F and 3G). A cherry-pick screening confirmed twenty eight of the candidate miRNA-mimics (Figure 3H). Of note, overexpression of miR-199b-5p induced the highest proliferative activity (Figure 3G). However, miR-199b is known to mitigate pathological remodeling and fibrosis and therefore was not considered for further investigation. It is to be noted that several pro-proliferating miRNAs identified in the high-throughput screening are members of miRNA-families and clusters, such as miR-148a-3p/miR-148b-3p (miRNA-148 family) and miR-212-3p/miR-132-3p (miRNA 212/132 family). Further of note, five candidate miRNAs belong to the imprinted chromosome 19 miR- cluster (C19MC; hsa-miR-515-3p, hsa-miR-519e-3p, hsa-miR-517c-3p) and adjacent miR-371-3 cluster (hsa-miR-371a-3p, hsa-miR-371b-3p), located on chromosome 19q13.41). This primate-specific cluster represents one of the largest gene cluster of human miRNAs. Members of the C19MC are expressed in the placenta and in undifferentiated cells. In human embryonic stem cells (hESCs), this cluster is markedly upregulated as compared to non-hESCs, indicating an important role for dedifferentiation.

Further, several miRNAs of C19MC directly target cyclin-dependent kinase inhibitor 1A (CDKN1A) at its 3'-untranslated region in HEK 293 cells. CDKN1A is an important cell cyclin-dependent kinase (CDK)- inhibitor that regulates cell proliferation and G1/S transition. Of note, members of the C19MC are also involved in extracellular matrix composition that is known to impact on regenerative capacity of the heart.

In conclusion, in placental-derived cells, miRNAs that belong to the C19MC cluster regulate proliferation, immunomodulation and extracellular matrix composition, biological features that are also highly relevant for cardiac regeneration. Therefore, the three C19MC-associated miRNAs with the highest proliferative activity in the high-throughput screening, namely hsa-miR-515-3p, hsa-miR-519e-3p and hsa-miR-371a-3p were further investigated for induction of human cardiomyocyte proliferation.

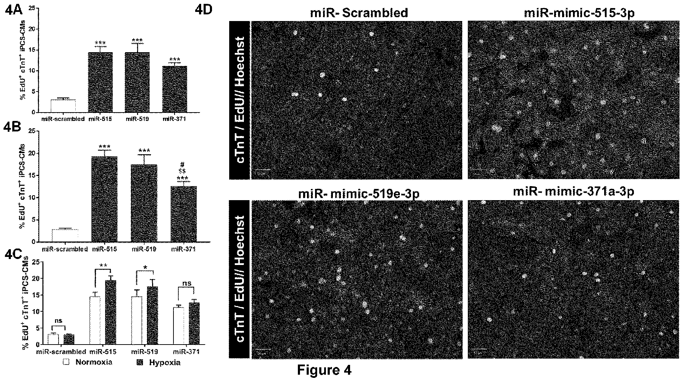

The findings derived from the high-throughput screening were evaluated and the results are shown in Figure 4:
Transfection of hiPSC-CMs with miR-515-3p, miR-519e-3p and miR-371a-3p-mimic substantially increased incorporation of EdU in human iPSC-CMs as compared to miR-scrambled transfected hiPSC-CMs (see Figure 4A). In addition, we exposed miR-mimic transfected hiPSC-CMs to transient hypoxia (0.5% O2 for 2 hours). A brief period of hypoxia further significantly increased proliferative activity after treatment with miR-515-3p and miR-519e-3p, that was not observed in miR-scrambled or miR-371- mimic transfected hiPSC-CMs (see Figure 4B and 4C).

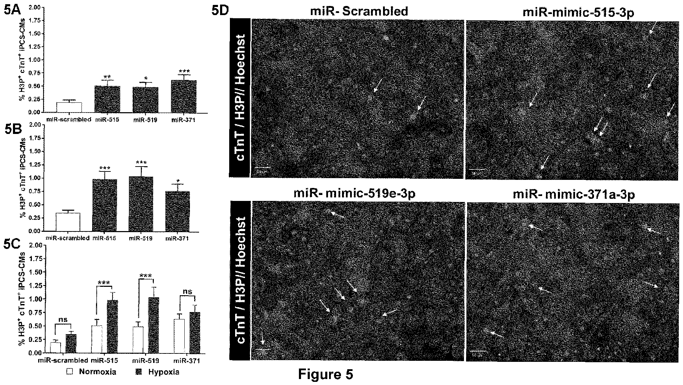

Furthermore, the mitosis marker histone H3P was analyzed. The results are shown in Figure 5:
Treatment with miR-515-3p, miR-519e-3p and miR-371a-3p-mimic increased H3P-positive hiPSC-CM as compared to miR-scrambled treated hiPSC-CMs (see Figure 5A). In addition, a transient period of hypoxia, as compared to normoxia, increased the number of H3P-positive hiPSC-CM in miR-519e-3p and miR-515-3p-mimic transfected hiPSC-CMs (see Figure 5B and 5C). These results indicate that miR-515-3p, miR-519e-3p and miR-371a-3p induce cardiomyocyte proliferation. As miR-515-3p and miR-519e-3p showed a more pronounced induction of proliferative activity under transient hypoxia as compared to miR-371a-3p, the further experiments focused on these two miRNAs.

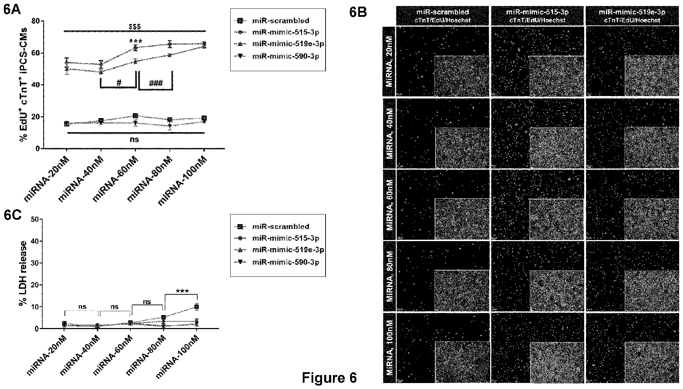

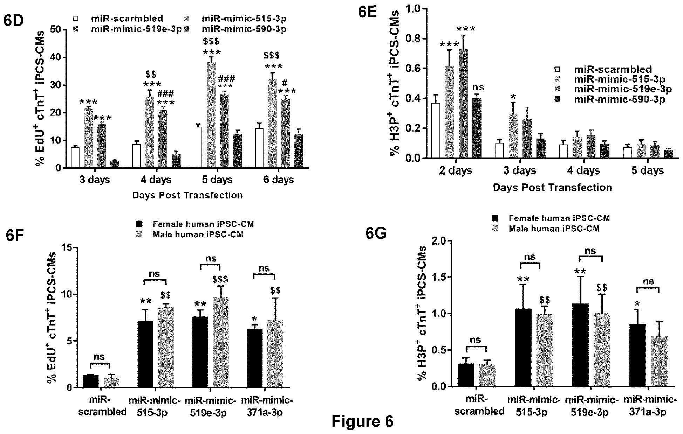

In order to define efficacy and toxicity effects, miRNAs were transfected with increasing concentrations, the results are shown in Figure 6.

Transfection with 60nM induced proliferative activity significantly above lower concentrations (see Figure 6A-C) without affecting cytotoxicity, as assessed by LDH assay (see Figure 6C). Of note, transfection of hsa-miR-590-3p that is known to induce cardiomyocyte proliferation in murine cardiomyocytes did not result in enhanced EdU incorporation in human iPSC-CM. EdU-incorporation and positive staining for H3P was analyzed over a period of six days after transfection (see Figure 6E). Whereas hiPSC-CMs showed highest EdU-incorporation at day five (see Figure 6D), H3P-positive hiPSC-CMs peaked at day two post transfection (see Figure 6E). Of note, miR-515-3p and miR-519e-3p-mimic transfection was effective in hiPSC-CMs from both, female or male donors (Figure 6F and 6G).

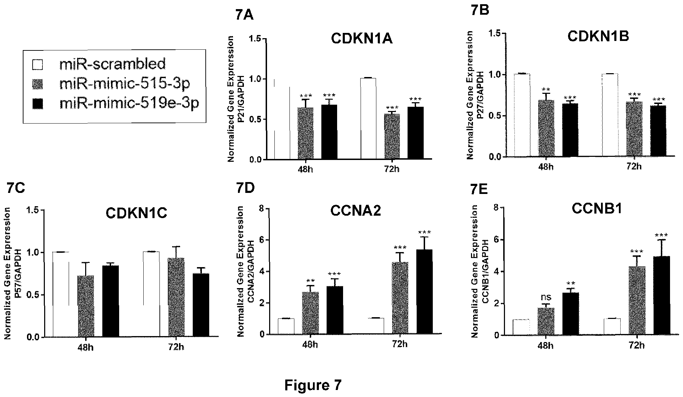

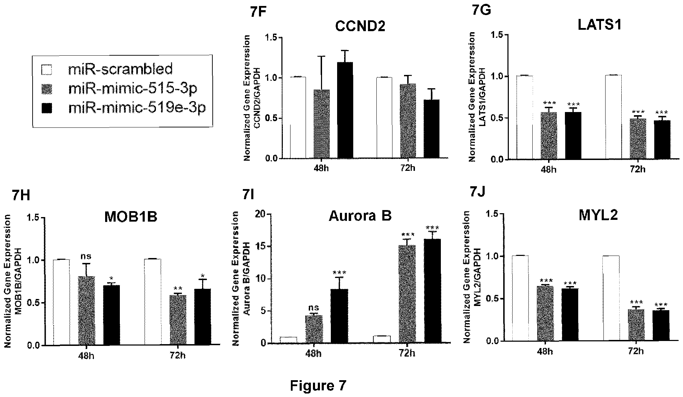

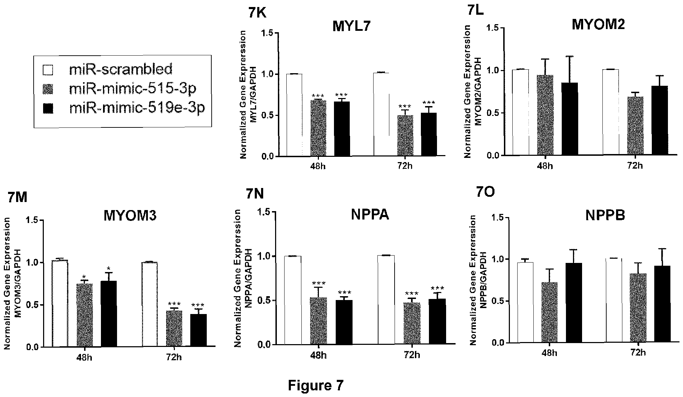

Cardiomyocyte proliferation is regulated by genes that are necessary for cell-cycle reentry. Therefore, positive and negative cell cycle regulators using RT-qPCR after transfection of hiPSC-CM with miR-515-3p and miR-519e-3p-mimic were assessed. The results are shown in Figure 7:
A marked decrease in expression of cyclin-dependent kinase inhibitor 1A (CDKN1A) after miR-515-3p and miR-519e-3p-mimic transfection was shown. Similarly, downregulation of CDKN1B was observed. Importantly, miR-515-3p and miR-519e-3p-mimic-transfected hiPSC-CMs showed a substantial increase in Cyclin A2 (CCNA2) expression. It is known that Cyclin A2 is a key regulator of cell cycle through G1-S and G2-M phase. In addition, the cell-cycle activator Cyclin B1 (CCNB1) was markedly upregulated, whereas Cyclin D2 (CCND2) did not show a differential expression. Furthermore, genes that are involved in the Hippo-signaling pathway, such as LATS1 and MOB1B were significantly downregulated after miR-515-3p and miR-519e-3p-mimic transfection. In line, sarcomeric genes that are involved in structural maturation, such as Myosin light chain-2 (MYL-2), Myosin light chain-7 (MYL-7) and Myomesin 3 (MYOM-3) were markedly downregulated. As upregulation of cycling markers and downregulation of dedifferentiation markers may be associated with a hypertrophic response and adult cardiomyocyte remodeling, Natriuretic Peptide A (NPPA) and Natriuretic Peptide B (NPPB) were assessed. Importantly, NPPA was significantly downregulated after miR-515-3p and miR-519e-3p-mimics transfection and NPPB did not show altered expression, indicating miRNA-dependent cell-cycle reentry in the absence of hypertrophic growth.

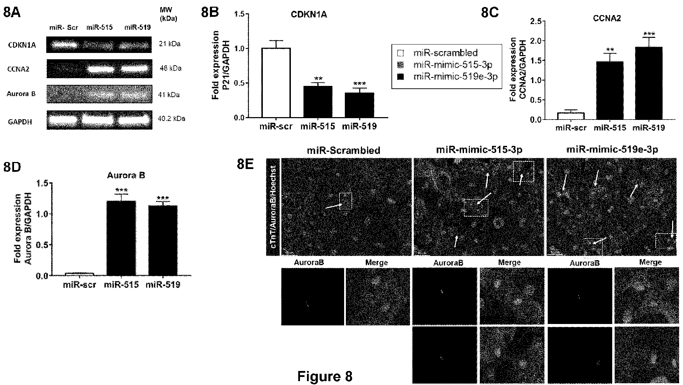

The findings were evaluated by conducting Western blots. The results are shown in Figure 8:
After transfection of miR-515-3p, miR-519e-3p-mimics in hiPSC-CMs, a marked downregulation of cell-cycle inhibitor CDKN1A and upregulation of the positive cell-cycle regulator Cyclin A2 (CCNA2) on protein level was confirmed (see Figure 8A-8D). In addition, Aurora B (AURKB), a midbody associated protein marker needed for completion of cell division, was significantly upregulated. Immunofluorescence staining further confirmed localization of Aurora B in the midzone of dividing hiPSC-CMs (see Figure 8E).

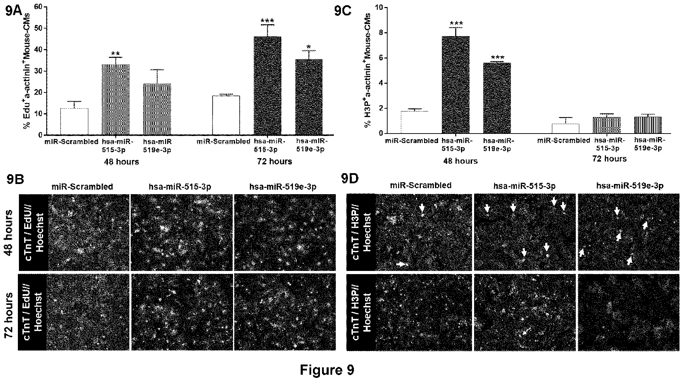

Notably, the investigated miRNAs - miR-515-3p and miR-519e-3p - are primate- specific and therefore not expressed in mammals. To show that miR-515-3p and miR-519e-3p are also relevant for cardiomyocytes proliferation in non-primate cardiomyocytes, hsa-miR-515-3p and hsa-miR-519e-3p were tested in mouse cardiomyocytes. The results are shown in Figure 9:
Cardiomyocytes were isolated from mice at day three after birth (P3). In line with our findings in human iPSC-CM, transfection of P3 mouse cardiomyocytes with miR-515-3p and miR-519e-3p-mimics markedly enhanced incorporation of EdU (Figure 9A, B) and H3P (Figure 9C, D). Together, these data indicate that human miR-515-3p and miR-519e-3p substantial induction of cardiomyocyte proliferation is not restricted to primates.

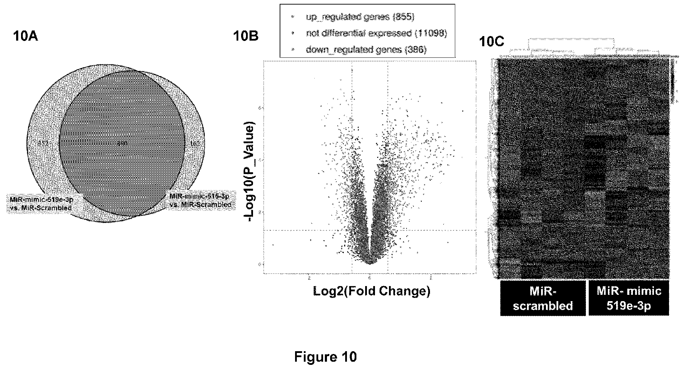

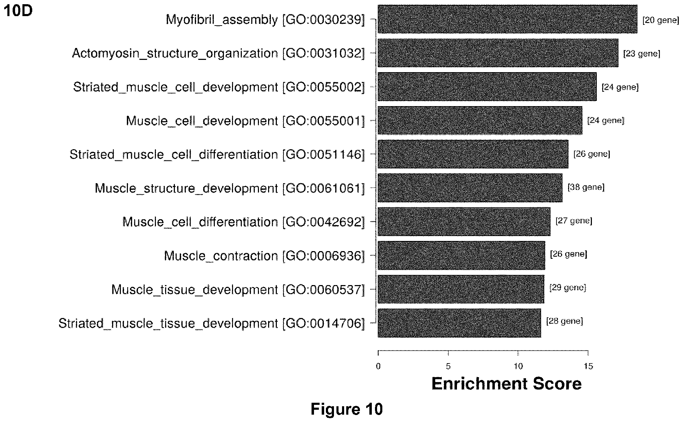

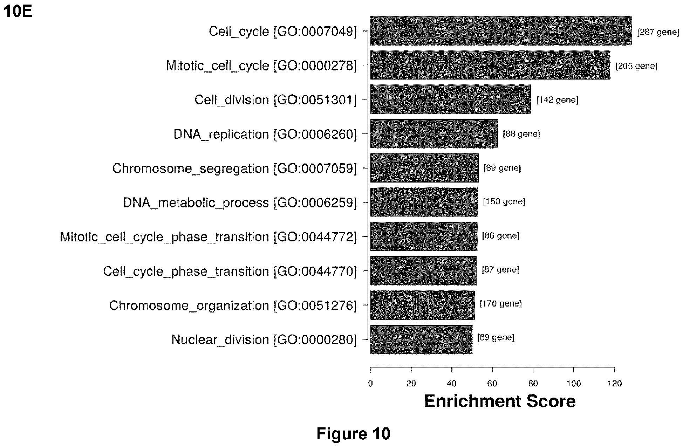

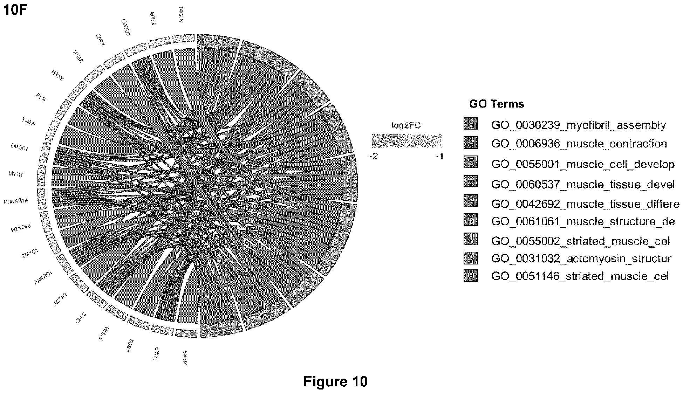

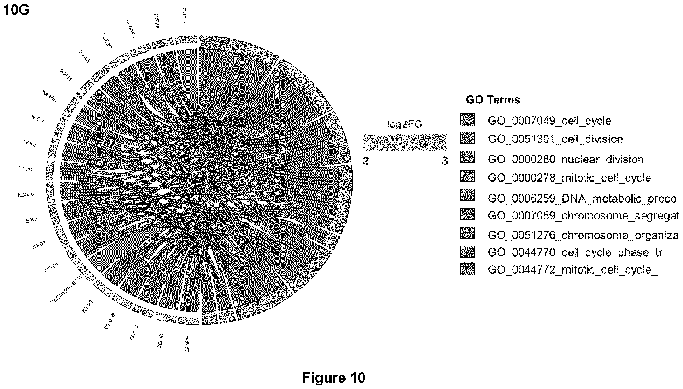

To further understand pathways involved in the observed regenerative potential, RNA-Sequencing (RNA-Seq) was performed after overexpression of miR-515-3p and miR- 519e-3p in hiPSC-CM. The results are shown in Figure 10:
As expected, regulated genes after miR-515-3p and miR-519-3p-mimic transfection show an extensive overlap, with 890 shared regulated genes (see Figures 10 A).

Gene ontology (GO) enrichment analysis was used to investigate whether specific GO terms are associated with differentially expressed genes after miR-519-3p mimic treatment (see Figures 10 D-G). Downregulated gene pathways are strongly involved in sarcomeric organization signaling (see Figure 10 D, F). In contrary, upregulated enriched gene pathways comprise processes involved in cell-cycle regulation, specifically mitosis, cell division, nuclear division, DNA replication and chromosome assembly (see Figure 10 E, G). As expected, cell cycle genes such as Cyclins (CCNB1, CCNB2, CCNA2), Kif family members (KIF4a, KIF20A, KIFC1, KIF2C, KIF11) and cytokinesis genes such as Aurora (AURKA, AURKB) and NUSAP1belong to the most significantly upregulated genes derived from the RNA-Seq (see Figure 10 G). Notably, most of the genes that are regulated by YAP are upregulated after miR-515 or miR-519e-3p overexpression in human iPSC-CMs, such as ANLN, BIRC5, CDC20, CENPF, ECT2GAS2L3, KIF23, NUSAP1, TOPA2. In addition, genes that are mandatory for coordinated cytokinesis resulting in cell division such as Anillin (ANLN), IQGAP3 and GAS2L3 were highly upregulated. These findings indicate that miR-515-3p of miR-519e-3p strongly induce signaling pathways involved in cardiomyocyte proliferation.

In the following section, the results with regard to Figures 11, 12 and 13 will be discussed:
A marker - H3P - was used that specifically identifies cells in mitosis. Notably, human iPSC-CM that are transfected with miR-mimic-515-3p and miR-mimic-519e-3p have a substantial increase in H3P-positive hiPSC-CM as compared to miR-scrambled transfected (control) hiPSC-CM. For identification of hiPSC-CM undergoing cytokinesis after miR-515-3p, miR-519e-3p and miR-scrambled transfection, Aurora B-kinase was visualized. Herein, it was possible to differentiate all stages of late mitosis, including movement of Aurora B-kinase to the midbody in cytokinesis (see Fig. 11A). In addition, miR-515-3p and miR-519e-3p treated hiPSC-CMs showed disorganization of sarcomere structures (see Fig. 11B). Importantly, the number of hiPSC-CM in prophase or stages of late mitosis were substantially increased after miR-515-3p and miR-519e-3p transfection, as indicated by Aurora B-kinase positive nuclei (Fig. 11C). Further, a substantially higher number of cells positive for localization of Aurora B-kinase in the midbodies was observed indicating cytokinesis in hiPSC-CMs after treatment with miR-515-3p and miR-519e-3p. (see Fig. 11D-E). In conclusion, these results convincingly show that miR-515-3p and miR-519e-3p substantially induce early and late stages of mitosis and cell division.

Of note, miRNA that have been identified using EdU-incorporation do not necessarily induce mitosis and proliferation. Importantly, transient hypoxia does involve a critical step to decipher miRNAs that not only increases EdU, a marker of DNA-synthesis, but also mitosis and cytokinesis, leading to cell division. Therefore, hypoxia has not only been used for mimicking hypoxia-reoxygenation that occurs in the setting of myocardial infarction, but also to reveal miRNAs with a robust increase in proliferation. In this regard, it was shown that hypoxia can roughly double the increase of mitosis in hiPSC-CM after miR-mimic-515-3p and miR-mimic-519e-3p transfection as compared to other miRNAs, such as miR-371a-3p that did not increase mitosis in hiPSC-CM after hypoxia.

All in all, it was surprisingly shown that miRNAs, specifically miR-515-3p and miR-519e-3p can enhance mitosis and cytokinesis after transient transfection in hiPSC-CM.

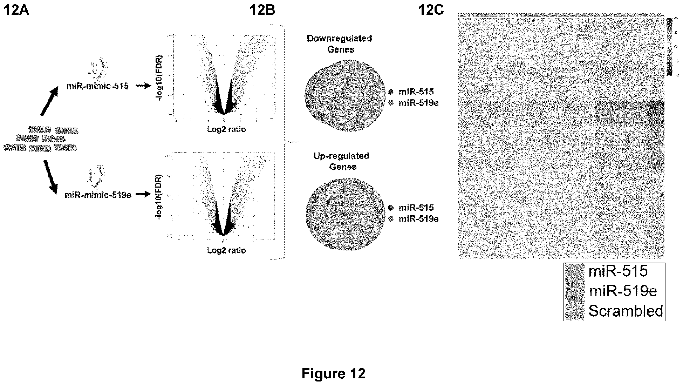

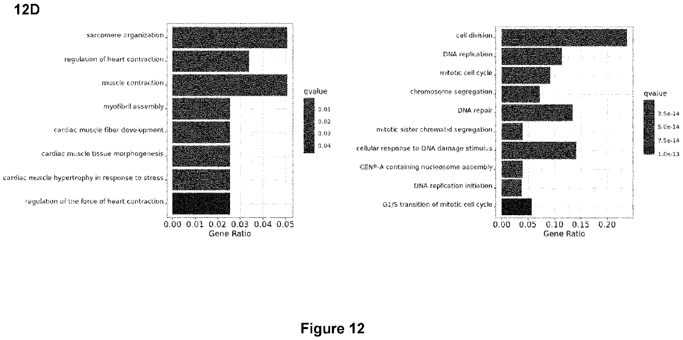

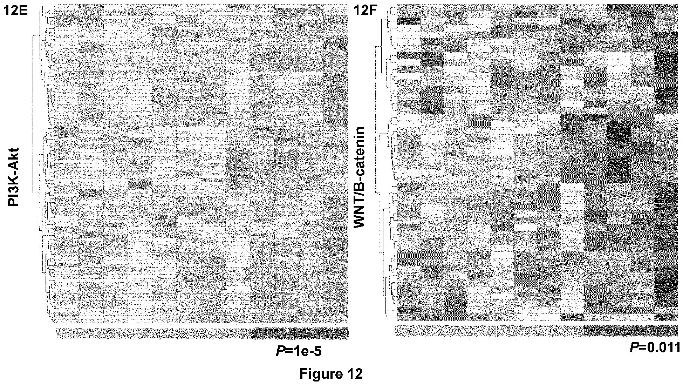

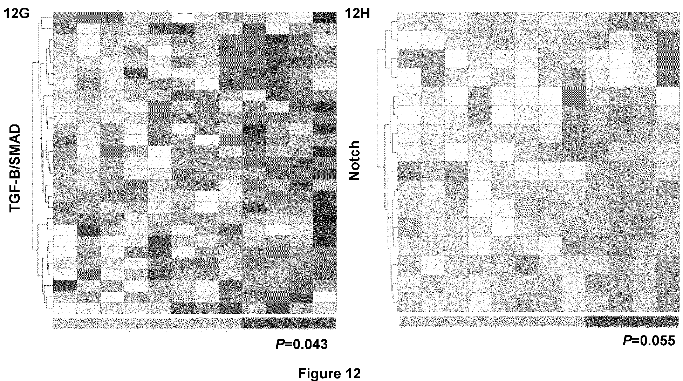

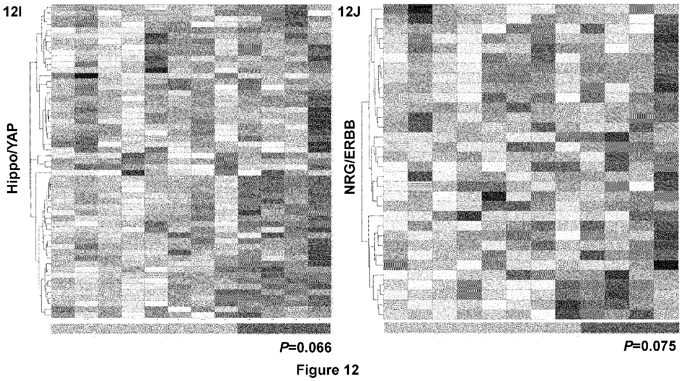

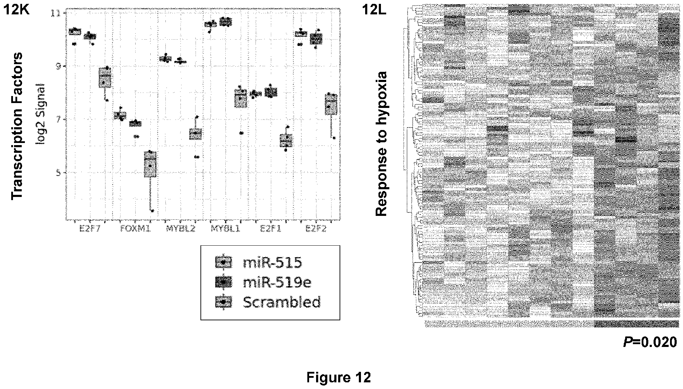

RNA-Seq analysis in hiPSC-CM cardiomyocytes after overexpression of miR-515-3p and miR-519e-3p show that these miRNA modulate the PI3K-Akt, WNT/B-catenin and TGF-Beta/SMAD pathways (see Fig. 12 E-G) but not significantly the Hippo/YAP pathway (see Fig. 12I). Moreover, transcription factors from the E2F family (E2F1, E2F2 and E2F7) and FOXM1 that are important for initiation of cell cycle and that were reported to fail to be reactivated in mature cardiomyocytes upon myocardial infarction were upregulated upon miR-mimic-515-3p and -519e-3p transfection.

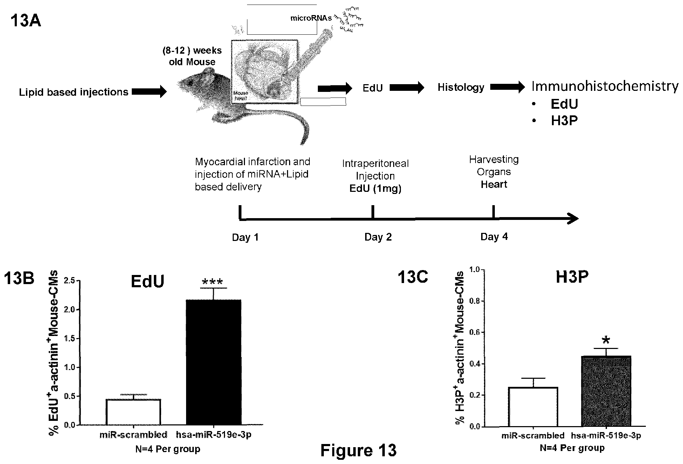

Furthermore, it was shown that miR-515-3p and miR-519e-3p can induce cytokinesis in hiPSC-CM. Furthermore, it was tested if miR-519e-3p can induce cardiomyocyte proliferation in vivo. A myocardial infarction model performing permanent ligation of the left anterior descending (LAD) artery in adult (8-12 weeks) mice was used (see Fig. 13A). Histological analysis was performed three days after LAD ligation and intramyocardial injection of miR-mimic-519e-3p and miR-scrambled. The effect of adult cardiomyocyte proliferation in vivo was assessed using EdU (see Fig. 13B) and H3P (see Fig. 13C). A significant increase in EdU-positive cardiomyocytes in miR-mimic-519e-3p treated mice as compared to miR-scrambled, indicative of induction of cell cycle activation was found.

Strikingly, a significant increase of H3P-positive cardiomyocytes was observed, indicating that miR-519e-3p induce mitosis of cardiomyocyte in vivo (see Fig. 13C). These data show that miR-519e-3p not only induce cytokinesis of hiPSC-CM in vitro, but also increase cardiomyocyte proliferation in adult cardiomyocytes in vivo.

### Discussion:

In the following a short summary of the above-presented results is given:
By the above-presented results, it is shown that primate-specific miRNA hsa-miR-515-3p and hsa-miR-519e-3p induce cell-cycle re-entry in human iPSC-derived cardiomyocytes. A functional high-throughput screening in human iPSC-CMs to assess the proliferative capacity after hypoxia/reoxygenation using a miRNA library consisting of 2019 miRNA-mimics and anti-miRNAs was used for individual overexpression and downregulation of miRNAs in human iPSC-CM. Overexpression of 28 miRNAs (miRNA-mimics) induced cell-cycle reentry. Conversely, none of the anti-miRNAs yielded a robust proliferative response in human iPSC-CM. Of note, whereas some of the miRNAs that were reported to induce cell-cycling in murine cardiomyocyte were confirmed in our screening, such as miR-1825 and miR-33b, others did not show a significant proliferative effect in human iPSC-CMs after transient miRNA-overexpression, such as miR-590 and miR-199a, the miR-302/367 cluster and the miR-17-92 family. These findings suggest that proliferative response to miRNA-mimic treatment between human and murine-derived cardiomyocytes underlies different regulation. Intriguingly, five (miR-515- 3p, miR-519e-3p, miR-517c-3p and miR-371a-3p, miR-371b-3p) out of 28 miRNA-candidates derived from the screen belong to the primate-specific chromosome 19 miR-cluster (C19MC), that is expressed predominantly in placental tissue and adjacent miR-371-3 cluster. Experimental and clinical studies revealed that miRNA-members of the C19MC are involved in biological processes relating to proliferation, migration, differentiation, extracellular matrix composition and immunomodulation. Thereby, it is comprehensible that these miRNAs are important for cardiomyocyte proliferation due to their involvement of biological processes that are key factors for cardiac regeneration.

It was shown that miR-515-3p and miR-519e-3p substantially enhance expression of cell-cycling markers in human iPSC-CM. Importantly, transient hypoxia further increased incorporation and labeling of early and late mitosis markers. In addition, Aurora B, a protein kinase mandatory for cytokinesis, was strongly upregulated in miR-515-3p and hsa-miR-519e-3p-mimic treated human iPSC-CMs. As experimental studies suggest that a distinct population of adult cardiomyocytes maintain their proliferative capacity in a hypoxic environment and activation of cell-cycle in cardiomyocytes is more prominent at the borderzone of the infarcted myocardium, miR-515-3p and miR-519e-3p may enhance cardiogenesis in patients with myocardial infarction and heart failure. To further understand the underlying mechanism, common targets involved in the induction of cell-cycling were investigated. Cyclin-dependent kinase inhibitors CDKN1A and CDKN1B were significantly downregulated and, as a consequence, a substantial increase in the expression for cyclins (Cyclin A2 and Cyclin B1) was observed.

Furthermore, dedifferentiation markers, as assessed by RT-qPCR, were downregulated in the absence of upregulation of hypertrophic markers (NPPA, NPPB), suggesting that miR-515-3p and miR-519e-3p induce proliferation without hypertrophic response. Using an RNA-Seq approach, it was further observed that genes involved in sarcomeric organization pathways are strongly downregulated, most probably reflecting sarcomeric disassembly that is needed for cell division. Importantly, besides an upregulation of cyclins and genes involved in the Hippo pathway, a strong upregulation of genes that are involved in cytokinesis, cell abscission and cell division was observed.

This compelling evidence suggests that miR-515-3p and miR-519e-3p used as a medicament according to the present invention can force human cardiomyocytes to divide and therefore be used as a potential therapy for cardiac regeneration.

In addition to the foregoing explanations, the following enumerated aspects 1 to 12 are also relevant for the present disclosure as part of the specification which must not be confused with the appended claims:
According to a first aspect of the present disclosure a miRNA is disclosed comprising or consisting of a RNA sequence having at least 80% sequence homology, preferably 90% sequence homology, most preferably 100% sequence homology with one of the following miRNA sequences:
hsa-miR-515-3p with SEQ ID NO: 1, and
hsa-miR-519e-3p with SEQ ID NO: 2,
or a member of the miR-517 family, specifically hsa-miR-517c-3p with SEQ ID NO: 3 and/or hsa-miR-517a-3p with SEQ ID NO: 4,
or a primary transcript thereof, a precursor thereof, a mimic thereof or a combination thereof for use as a medicament.

According to an second aspect of the present disclosure an miRNA for use according to the first aspect is disclosed, wherein the miRNA is for use in treatment of a cardiac disease associated with heart failure and/or loss of cardiac myocytes.

According to a third aspect of the present disclosure an miRNA for use according to the second aspect is disclosed, wherein the cardiac disease is selected from myocardial infarction, ischemic and non-ischemic cardiomyopathy, congestive heart failure, hypertrophic cardiomyopathy, dilated cardiomyopathy, myocarditis, acute coronary syndrome and heart failure.

According to a fourth aspect of the present disclosure an miRNA for use according to anyone of aspects 1 to 3 is disclosed, wherein the miRNA is for administration via gene therapy, and/or a (cardiotropic) virus , or a polymeric vector or a dendrimer-based vector or an inorganic or lipid nanoparticle or cell-derived membrane vesicles or scaffold-based delivery systems (hydrogels, electrospun fibers).

According to an fifth aspect of the present disclosure an miRNA for use according to anyone of aspects 1 to 4 is disclosed, wherein the miRNA is for oral, parenteral, perilingual or intravenous, intraarterial or intracoronary administration, preferably via a stent.

According to a sixth aspect of the present disclosure a vector for use in treatment of a cardiac disease associated with loss of cardiac myocytes is disclosed, wherein said vector comprises at least a miRNA for use according to anyone of aspects 1 to 5, a DNA coding for at least one of said miRNA for use according to anyone of aspects 1 to 5 or a combination thereof.

According to a seventh aspect of the present disclosure the vector for use according to the sixth aspect is an adeno-associated vector (AAV), in particular anyone of AAV1, AAV2, AAV6, AAV8, AAV9, AAV10 or a retrovirus, or a lentivirus.

According to an eighth aspect of the present disclosure a pharmaceutical composition comprising at least a miRNA is disclosed for use according to anyone of aspects 1 to 5 or a vector according to aspects 6 or 7, and at least one pharmaceutically acceptable excipient.

According to an ninth aspect of the present disclosure the pharmaceutical composition of aspect 8, wherein the pharmaceutical composition is for use in treatment of a cardiac disease associated with heart failure and/or loss of cardiac myocytes .

According to an tenth's aspect of the present disclosure the pharmaceutical composition of aspect 9, wherein the cardiac disease is selected from myocardial infarction, ischemic and non-ischemic cardiomyopathy, heart failure, hypertrophic cardiomyopathy, dilated cardiomyopathy, myocarditis.

According to an eleventh's aspect of the present disclosure a method for the in vitro expansion of cardiomyocytes derived from stem cells or adult cardiomyocytes is disclosed, the method comprising the step of contacting said cells with at least one miRNA for use according to anyone of aspects 1 to 5.

According to an twelfth's aspect of the present disclosure a method for stimulating proliferation of cardiomyocytes in vitro is disclosed, the method comprising the step of contacting said cells with at least one miRNA for use according to anyone of aspects 1 to 5.

## Claims

1. A miRNA comprising or consisting of a RNA sequence with the following miRNA sequence:
hsa-miR-4784 with SEQ ID NO: 6,
or a member of the miR-4784 family,
or a primary transcript thereof, a precursor thereof, a mimic thereof or a combination thereof for use as a medicament in treatment of a cardiac disease associated with heart failure and/or loss of cardiac myocytes.

2. An miRNA for use according to claim 1, **characterized in that** the cardiac disease is selected from myocardial infarction, ischemic and non-ischemic cardiomyopathy, congestive heart failure, hypertrophic cardiomyopathy, dilated cardiomyopathy, myocarditis, acute coronary syndrome and heart failure.

3. An miRNA for use according to anyone of claims 1 or 2, **characterized in that** the miRNA is for administration via gene therapy, and/or a (cardiotropic) virus , or a polymeric vector or a dendrimer-based vector or an inorganic or lipid nanoparticle or cell-derived membrane vesicles or scaffold-based delivery systems (hydrogels, electrospun fibers).

4. An miRNA for use according to anyone of claims 1 to 3, **characterized in that** the miRNA is for oral, parenteral, perilingual or intravenous, intraarterial or intracoronary administration, preferably via a stent.

5. A vector for use as a medicament, preferably for use in treatment of a cardiac disease associated with loss of cardiac myocytes, wherein said vector comprises at least a miRNA for use according to anyone of claims 1 to 4, a DNA coding for at least one of said miRNA for use according to anyone of claims 1 to 4 or a combination thereof.

6. The vector for use according to claim 5, **characterized in that** said vector is an adeno-associated vector (AAV), in particular anyone of AAV1, AAV2, AAV6, AAV8, AAV9, AAV10 or a retrovirus, or a lentivirus.

7. A pharmaceutical composition comprising at least a miRNA for use according to anyone of claims 1 to 4 or a vector according to claims 5 or 6, and at least one pharmaceutically acceptable excipient.

8. The pharmaceutical composition of claim 7, wherein the pharmaceutical composition is for use in treatment of a cardiac disease associated with heart failure and/or loss of cardiac myocytes .

9. The pharmaceutical composition of claim 8, wherein the cardiac disease is selected from myocardial infarction, ischemic and non-ischemic cardiomyopathy, heart failure, hypertrophic cardiomyopathy, dilated cardiomyopathy, myocarditis.

10. A method for the in vitro expansion of cardiomyocytes derived from stem cells or adult cardiomyocytes, the method comprising the step of contacting said cells with at least one miRNA for use according to anyone of claims 1 to 4.

11. A method for stimulating proliferation of cardiomyocytes in vitro, the method comprising the step of contacting said cells with at least one miRNA for use according to anyone of claims 1 to 4.
